# EUROPEAN PATENT APPLICATION

(11) **EP 3 279 308 A1**
(43) Date of publication of application: **07.02.2018**
(21) Application number: 16771923.6
(22) Date of filing: 15.02.2016
(51) Int. Cl.: C12M 1/00, C12M 3/00

(54) **MEDICAL INSTRUMENT, FLUORINATED CYCLIC OLEFIN POLYMER, FLUORINATED CYCLIC OLEFIN POLYMER COMPOSITION, AND CELL CULTURE METHOD**

(30) Priority: 31.03.2015 JP 2015070868
(71) Applicant: Mitsui Chemicals, Inc., Tokyo 105-7117 (JP)
(72) Inventor: SUNAGA Tadahiro, Sodegaura-shi Chiba 299-0265 (JP); ODA Takashi, Sodegaura-shi Chiba 299-0265 (JP); OHKITA Hisanori, Singapore 117406 (SG); MIYASAKO Hiroshi, Mobara-shi Chiba 297-0017 (JP)
(74) Representative: Hoffmann Eitle
(86) International application number: PCT/JP2016/054318
(87) International publication number: WO 2016/158039

(57) **Abstract**

Medical instrument of the present invention includes a substrate using a fluorine-containing cyclic olefin polymer containing a structural unit represented by General Formula (1), in which the substrate has one surface where the substrate comes into contact with cells, and the substrate is provided with a convex-concave structure on the one surface, a ratio (L1/L2) of a width (L1) between convexities formed by the convex-concave structure and a maximum diameter (L2) of inoculated cells per cell in the cells is 1 to 300, and the cells do not adhere to or attach to the one surface provided with the convex-concave structure and the medical instrument promotes cell proliferation. 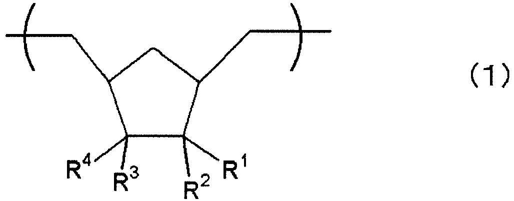 (in Formula (1), at least one of R¹ to R⁴ is fluorine, an alkyl group with 1 to 10 carbon atoms which contains fluorine, an alkoxy group with 1 to 10 carbon atoms which contains fluorine, or an alkoxyalkyl group with 2 to 10 carbon atoms which contains fluorine, and, when R¹ to R⁴ are groups which do not contain fluorine, R¹ to R⁴ are selected from hydrogen, an alkyl group with 1 to 10 carbon atoms, an alkoxy group with 1 to 10 carbon atoms, or an alkoxyalkyl group with 2 to 10 carbon atoms, R¹ to R⁴ may be the same as or different from each other, and R¹ to R⁴ may be bonded to each other to form a cyclic structure.)

## Description

### TECHNICAL FIELD

The present invention relates to medical instrument, a fluorine-containing cyclic olefin polymer, a fluorine-containing cyclic olefin polymer composition, and a cell culture method.

### BACKGROUND ART

Many flora and fauna cells have been cultured in the related art and various types of culturing techniques have been researched. Cell culturing techniques are techniques which are essential for, in particular, the development of medicine, the investigation of pathological mechanisms, and the like in the field of life science. In addition to cell culture techniques for research purposes, various types of industrial production culture methods have been researched for the purpose of use in fields such as biology, medical science, pharmaceutical science, and immunology. Furthermore, in recent years, in fields such as medicine, research has also been actively carried out for culturing tissue cells and using these as substitute tissues for artificial organs, artificial dentary bone, artificial skin, and the like.

Such cell culturing is normally performed with a culture solution in a certain container. Here, many animal cells have adhesion-dependency and are attached to a substance to be grown and a substrate (instrument) for attaching the cells is necessary for culturing the cells which have adhesion-dependency. As a substrate for cell culturing, one molded from polystyrene as a raw material is generally used. In addition, substrates on which a low-temperature plasma treatment, a corona discharging treatment, or the like are carried out on the surface of the substrate and to which hydrophilicity is imparted are commercially available as culturing instrument such as Petri dishes, flasks, and multi-plates.

However, it is known that, in a case where attaching cell types are cultured using culturing instrument, the cells can cover the culturing surface of the culture container, which can cause contact inhibition in which cell growth stops. In addition, a concentration effect is also known in which, when the concentration of the inoculated cells is excessively low, the adhesion or proliferation of cells is influenced even in an environment in which nutrients, oxygen, and the like are sufficiently supplied to the cells. In addition, an operation of repeatedly inoculating and culturing (referred to below simply as a subculture operation) is used when cell sheets, spheroids, and colonies are produced in a state in which cell proliferation applied to tissue culture forms a group. In this operation process, since the cells behave so as to proliferate in a planar manner while attaching to the culturing surface of the culture container, the operation becomes complicated, and there are cases where it is difficult to culture the cells with good reproducibility without damage.

In order to solve these problems, a method for producing cultured mucous membranes/skins by inoculating and culturing fibroblasts, keratinocytes, or the like by arranging collagen which is cross-linked in a gel or sponge form on a culture substrate is disclosed (refer to Patent Document 1) ; however, many of the collagen types which are used are collagens which are solubilized and extracted from the connective tissues of cows or pigs and, due to the recent problems such as bovine spongiform encephalopathy (BSE) or foot and mouth disease, the use of the above is becoming more problematic in cases where medical applications or the like are considered.

For the subculture operation, in the techniques in the related art, in order to culture attaching cells which divide repeatedly, a method in which, every time the proliferation reaches a target cell concentration after culturing the cells in a culture container for an appropriate period of time, an operation of separating cells from a culturing surface and transferring some of the cells into a new culture container is continuously performed, was typical (for example, refer to Patent Documents 2 and 3). Patent Document 2 discloses a method of carrying out culturing in a closed system while using a culture container which has a plurality of culturing surfaces with different areas, starting cell culturing from the culturing surface which has the smallest area, separating cells with a scraper as the proliferation progresses, and gradually moving the cells to a culturing surface with a large area. In addition, Patent Document 3 discloses a subculture operation in which the cell concentration is adjusted by connecting cell bags made of a resin with gas permeability and mixing a culture solution in the bags. However, in any of the methods, since the subculture operation involves extremely complicated work such as solution exchanges and an operation of peeling off the cells attached to the substrate and transferring these cells to a new culture container is also performed, there are potentially problems whereby the cells might be damaged and the proliferated form thereof destroyed, or the like.

With regard to the properties of the substrate surface on which cells are cultured and the proliferation property, the results of evaluating the relationship between the proliferated form of the cells and the water contact angle of the substrate are disclosed (refer to Non-Patent Document 1) using an example in which L cells (secretory cells from the stomach and intestines) were cultured. In this document, the correlation between the contact angle with respect to water and the adhesion of cells using various substrates is investigated. In addition, in this investigation, it is shown that a substrate having a contact angle with respect to water of 60° to 80° is excellent in L cell adhesion.

In addition, culturing techniques have recently been proposed which use substrates in which a convex-concave structure is formed on the surface of the substrate with which cells are in contact (for example, refer to Patent Documents 4 and 5). In many cases, culturing which uses a substrate provided with such a convex-concave structure is spheroid culturing and the cells exhibit an aspect of proliferating in an aggregated form. However, the material of the substrate provided with a convex-concave structure is a cell adhesion material known in the related art such as cyclic olefin polymer (refer to Patent Document 4), polydimethylsiloxane (refer to Patent Document 5), or the like and, in spheroid culturing using the convex-concave structure of the substrate, even when the area in which cells are in contact with the substrate is small, the form of the adhesion substantially does not change and cells form an anchorage. Due to this, there is a potential problem in that, when detaching the cultured cells from the substrate, the cells maybe damaged and the proliferated form thereof destroyed. Here, while reports have been made regarding the effective shape and size of the convex-concave structure when the cells form the anchorage in cell culturing which uses a substrate provided with a convex-concave structure, there has been no description of the relationship between the water contact angle of a substrate surface and the adhesion or the proliferation property.

### RELATED DOCUMENT

### PATENT DOCUMENT

[Patent Document 1] Japanese Unexamined Patent Publication No. 2004-147585
[Patent Document 2] Japanese Unexamined Patent Publication No. 2004-129558
[Patent Document 3] Japanese Unexamined Patent Publication No. H07-047105
[Patent Document 4] Pamphlet of International Publication WO2007/097121
[Patent Document 5] Japanese Unexamined Patent Publication No. 2010-88347

### NON-PATENT DOCUMENT

[Non-Patent Document 1] Y. Tamada, Y. Ikeda, Journal of Colloid and Interface Science 155, 334-339 (1993)

### SUMMARY OF THE INVENTION

### TECHNICAL PROBLEM

The present invention has an object of providing medical instrument which makes it possible to detach cells in contact with or held on a surface of a substrate from the substrate without damaging the cells.

### SOLUTION TO PROBLEM

The present invention is shown below.
(1) Medical instrument comprising a substrate using a fluorine-containing cyclic olefin polymer containing a structural unit represented by General Formula (1), in which the substrate has one surface where the substrate comes into contact with cells, and the substrate has a convex-concave structure on the one surface, a ratio (L1/L2) of a width (L1) between convexities formed by the convex-concave structure and a maximum diameter (L2) of inoculated cells per cell in the cells is 1 to 300, and the cells do not adhere to or attach to the one surface provided with the convex-concave structure and the medical instrument promotes cell proliferation. 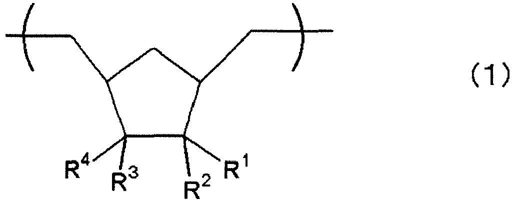 (in Formula (1), at least one of R¹ to R⁴ is fluorine, an alkyl group with 1 to 10 carbon atoms which contains fluorine, an alkoxy group with 1 to 10 carbon atoms which contains fluorine, or an alkoxyalkyl group with 2 to 10 carbon atoms which contains fluorine, and, when R¹ to R⁴ are groups which do not contain fluorine, R¹ to R⁴ are selected from hydrogen, an alkyl group with 1 to 10 carbon atoms, an alkoxy group with 1 to 10 carbon atoms, or an alkoxyalkyl group with 2 to 10 carbon atoms, R¹ to R⁴ may be the same as or different from each other, and R¹ to R⁴ may be bonded to each other to form a cyclic structure.)
(2) The medical instrument according to (1), in which the L1/L2 is 1 to 100.
(3) The medical instrument according to (1) or (2), in which a width between convexities of the convex-concave structure is 10 *µ*m to 1,000 *µ*m.
(4) The medical instrument according to any one of (1) to (3), in which the one surface has a water contact angle of 70° to 160°.
(5) The medical instrument according to any one of (1) to (4), in which the one surface is formed by a fluorine-containing cyclic olefin polymer composition including the fluorine-containing cyclic olefin polymer, a photocurable compound, and a photo-curing initiator.
(6) The medical instrument according to (5), in which a mass ratio (fluorine-containing cyclic olefin polymer/photocurable compound) of the fluorine-containing cyclic olefin polymer and the photocurable compound in the fluorine-containing cyclic olefin polymer composition is from 99.9/0.1 to 50/50.
(7) The medical instrument according to any one of (1) to (6), which is used for culturing cells in contact with the one surface.
(8) The medical instrument according to (7), in which the cultured cells from the cells float and proliferate while forming a group of growing cells selected from cell sheets, spheroids, or colonies.
(9) The medical instrument according to (7) or (8), in which a group of growing cells is liberated by a buffer solution and detached from the one surface.
(10) A fluorine-containing cyclic olefin polymer used, in medical instrument in which cells are brought into contact with one surface of a substrate having a convex-concave structure, for forming the one surface, the fluorine-containing cyclic olefin polymer comprising a structural unit represented by General Formula (1) below. 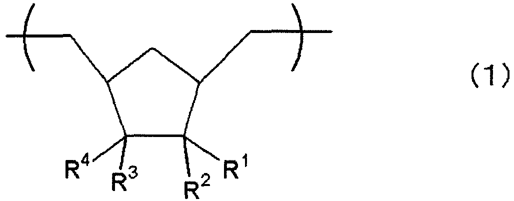 (in Formula (1), at least one of R¹ to R⁴ is fluorine, an alkyl group with 1 to 10 carbon atoms which contains fluorine, an alkoxy group with 1 to 10 carbon atoms which contains fluorine, or an alkoxyalkyl group with 2 to 10 carbon atoms which contains fluorine, and, when R¹ to R⁴ are groups which do not contain fluorine, R¹ to R⁴ are selected from hydrogen, an alkyl group with 1 to 10 carbon atoms, an alkoxy group with 1 to 10 carbon atoms, or an alkoxyalkyl group with 2 to 10 carbon atoms, R¹ to R⁴ may be the same as or different from each other, and R¹ to R⁴ may be bonded to each other to form a cyclic structure.)
(11) A fluorine-containing cyclic olefin polymer composition used, in medical instrument in which cells are brought into contact with one surface of a substrate having a convex-concave structure, for forming the one surface, the fluorine-containing cyclic olefin polymer composition comprising a fluorine-containing cyclic olefin polymer containing a structural unit represented by General Formula (1) below; a photocurable compound; and a photo-curing initiator. 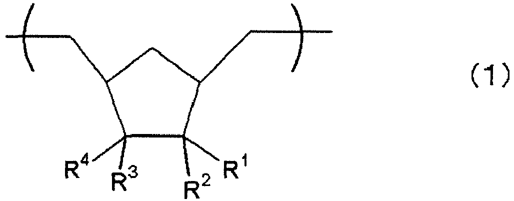 (in Formula (1), at least one of R¹ to R⁴ is fluorine, an alkyl group with 1 to 10 carbon atoms which contains fluorine, an alkoxy group with 1 to 10 carbon atoms which contains fluorine, or an alkoxyalkyl group with 2 to 10 carbon atoms which contains fluorine, and, when R¹ to R⁴ are groups which do not contain fluorine, R¹ to R⁴ are selected from hydrogen, an alkyl group with 1 to 10 carbon atoms, an alkoxy group with 1 to 10 carbon atoms, or an alkoxyalkyl group with 2 to 10 carbon atoms, R¹ to R⁴ may be the same as or different from each other, and R¹ to R⁴ may be bonded to each other to form a cyclic structure.)
(12) A cell culture method comprising a step of inoculating cells so as to contact one surface of the medical instrument according to any one of (1) to (9), a step of culturing the cells to obtain cultured cells, and a step of adding a buffer solution to the one surface to float cultured cells from the one surface.

### ADVANTAGEOUS EFFECTS OF INVENTION

According to the present invention, it is possible to provide medical instrument which makes it possible to detach cells which are in contact with or held on a surface of a substrate from the substrate without damaging the cells.

### BRIEF DESCRIPTION OF THE DRAWINGS

The object described above and other objects, features, and advantages will be made clearer from favorable embodiments which will be described below and the accompanying diagrams below.

FIG. 1 is a diagram showing a photomicrograph of light-and-dark images of mouse embryo fibroblasts floating in a sheet form with a phosphate-buffered physiological saline described in Example 1.
FIG. 2 is a diagram showing a fluorescent microscopy photograph of a cytoskeletal protein of human skin fibroblasts cultured for 14 days as described in Example 8.
FIG. 3 is a diagram showing a fluorescent microscopy photograph of a cytoskeleton protein of human skin fibroblasts cultured for 7 days as described in Comparative Example 4.

### DESCRIPTION OF EMBODIMENTS

Description will be given below of the present invention based on embodiments. In the present specification, "to" represents "equal to or more than A and equal to or less than B" unless otherwise specified.

### (Medical Instrument)

Medical instrument according to the present invention is shown below.

Medical instrument including a substrate using a fluorine-containing cyclic olefin polymer containing a structural unit represented by General Formula (1), in which the substrate has one surface where the substrate comes into contact with cells, and the substrate is provided with a convex-concave structure on the one surface, a ratio (L1/L2) of a width (L1) between convexities formed by the convex-concave structure and a maximum diameter (L2) of inoculated cells per cell in the cells is 1 to 300, and the cells do not adhere to or attach to the one surface provided with the convex-concave structure and the medical instrument promotes cell proliferation.

Here, the phrase "the cells do not adhere to or attach to the one surface provided with the convex-concave structure" means that when a culture solution or a buffer solution is added to one surface on which cultured cells are held, the cultured cells float and separate from the surface.

In addition, in the present embodiment, the cells to be brought into contact with one surface of the substrate may include stem cells.

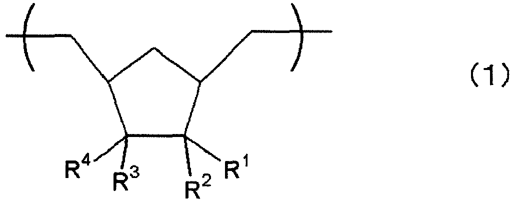

(In Formula (1), at least one of R¹ to R⁴ is fluorine, an alkyl group with 1 to 10 carbon atoms which contains fluorine, an alkoxy group with 1 to 10 carbon atoms which contains fluorine, or an alkoxyalkyl group with 2 to 10 carbon atoms which contains fluorine. R¹ to R⁴ are selected from hydrogen, an alkyl group with 1 to 10 carbon atoms, an alkoxy group with 1 to 10 carbon atoms, or an alkoxyalkyl group with 2 to 10 carbon atoms in a case where R¹ to R⁴ are groups which do not contain fluorine. R¹ to R⁴ may be the same as or different from each other. In addition, R¹ to R⁴ may be bonded to each other to form a cyclic structure. Preferably, each of R¹ to R⁴ in Formula (1) is a substituent which includes fluorine or a fluorine atom.)

The present inventors found that using a substrate forming a convex-concave structure formed of a fluorine-containing cyclic olefin polymer containing a structural unit represented by General Formula (1) or of a composition including the fluorine-containing cyclic olefin polymer on one surface of a substrate forming medical instrument in contact with or holding cells and bringing the inoculated cells into contact with or holding the inoculated cells on at least the bottom surface of a concave portion of the convex-concave structure makes it possible to appropriately and homogenously maintain the concentration of the cells over the entire surface of the substrate.

In addition, the present inventors found that, in the form after culturing, it is possible to form cell clusters having a homogenously cell concentration or groups such as cell sheets, spheroids, and colonies of homogenously thickness, and to easily float the cells held on the surface using a buffer solution such as phosphate-buffered physiological saline. Due to this, it is possible to detach the growing cells from the substrate without damaging the growing cells.

A detailed description will be given below of the medical instrument in the present invention.

The medical instrument in the present invention is provided with a substrate having a convex-concave structure and used in a form in which cells and a culture solution are brought into contact with or held on at least the bottom surface of a concave portion of one surface of the substrate. Such medical instrument may be for the purpose of culturing and proliferating cells which are brought into contact with or held on at least the bottom surface of a concave portion of one surface of the substrate on which the convex-concave structure is formed, to utilize the growing cells, or may be used to perform an inspection using the growing cells in contact with or held on at least the bottom surface of a concave portion of one surface of the substrate on which the convex-concave structure is formed.

On the other hand, in medical instrument for culturing cells, it is also possible to use at least the bottom surface of a concave portion of the one surface on which the convex-concave structure is formed as a culturing surface for culturing the cells.

In the medical instrument in the present invention, one surface on which cells of a substrate are in contact or held is formed of a fluorine-containing cyclic olefin polymer which contains a structural unit which is represented by General Formula (1). Due to this, medical instrument may be obtained which, for cells in contact with or held on one surface of a substrate for medical use or cell culturing or for cells cultured on one surface, is able to float a group of growing cells from a substrate by using a buffer solution such as phosphate-buffered physiological saline. Due to this, it is possible to detach the growing cells which are brought into contact with or held on one surface of the substrate on which the convex-concave structure is formed from the substrate without damaging the growing cells.

Furthermore, forming one surface of the substrate with the fluorine-containing cyclic olefin polymer containing the structural unit represented by General Formula (1) makes it possible to realize a substrate in which cells in contact with or held on one surface are not adhered to or attached to the one surface. Due to this, it is possible to separate cell culturing into cell sheets in which cells are proliferated while forming groups in the thickness direction, spheroids in which groups are proliferated individually and separately, and colonies in which groups are randomly dispersed.

In medical instrument which is used in order to culture normal cells, since the proliferated cells proliferate while adhered or attached to a culturing surface, the cells proliferate in a homogenously planar state with respect to the thickness direction, an individually separated spherical state, or a randomly dispersed colony state. In this case, since there is no longer an anchorage to which the cells are adhered or attached when the cells cover the culturing surface, cell growth is prevented due to the influence of contact inhibition or the like. On the other hand, it is possible to consider using a subculture operation which carries out culturing by repeating inoculation in order to efficiently carry out the cell culturing; however, there is a concern that cells may be damaged by work in which the cells are peeled off and transferred to a new culture container. In addition, the subculture operation involves extremely complicated work such as frequent solution exchanges.

In contrast to this, according to the present invention, forming a surface formed with a convex-concave structure of a substrate on which cells are in contact or held using a fluorine-containing cyclic olefin polymer containing the structural unit represented by General Formula (1) makes it possible to suppress the proliferated cells from being adhered or attached to the one surface of the substrate. For this reason, even without a subculture operation, it is possible to proliferate the cells so as to form a group in the thickness direction. Accordingly, it is possible to perform more effective cell culturing while suppressing damage to the cells and without complicating the operation.

In addition, in the medical instrument of the present invention, the ratio (L1/L2) of the width (L1) between convexities of the convex-concave structure formed by the fluorine-containing cyclic olefin polymer containing the structural unit represented by General Formula (1) and the maximum diameter (L2) of cells in contact with or held on the substrate is in the range of 1 to 300, preferably 2 to 200, and more preferably 3 to 100. Here, the maximum diameter (L2) of the cell indicates the maximum diameter per cell inoculated in the width between convexities (each concave portion). Here, L2 is a value obtained by measuring each of the maximum diameters of a plurality of cells inoculated in the width between convexities and averaging these values. Due to this, since it is possible to reliably bring the cells into contact with or hold the cells on the bottom surface of a concave portion and the cells grow within the individual partitions, the proliferation property of the cells is high due to appropriately and homogenously maintaining the concentration of the cells on the substrate surface and it is additionally possible to prepare cell clusters in group form having a homogenously cell concentration or cell sheets, spheroids, colonies, and the like having a homogenously thickness in the cultured form. Then, it is possible to easily float and detach cell clusters with a cultured group shape or cell sheets from a substrate using a buffer solution such as a phosphate-buffered physiological saline.

Here, the maximum diameter of the cells is a measurement value measured by microscopically observing a cell floating in a culture solution, and the size thereof varies depending on the type of cell. In general, most animal cells such as mouse, rat, human and the like are 10 *µ*m to 100 *µ*m, in particular, 10 *µ*m to 60 *µ*m in the case of human cells, and, depending on the type, approximately 2.5 *µ*m in sperm cells or 200 *µ*m in egg cells. Let the maximum diameter of the cell obtained from the measurement of the cell per microscopic observation be L2.

In accordance with this, the width between convexities of the convex-concave structure formed by the fluorine-containing cyclic olefin polymer containing the structural unit represented by General Formula (1) is preferably 10 *µ*m to 1,000 *µ*m. The width is more preferably 15 *µ*m to 1,000 *µ*m, and even more preferably 20 *µ*m to 1,000 *µ*m. Here, the width between convexities is a measured value when a pattern is observed from above, for example, diagonal in a pattern such as a square or a rectangle, a diameter in a circular pattern, and a maximum width in a pattern of an indefinite shape, and the shape is not particularly limited. Within this range, it is possible to bring the cells in contact with, or hold the cells on, the bottom surface of a concave portion of the convex-concave structure effectively, and, in the cultured form as described above, it is possible to prepare cell clusters of homogenously concentration, cell sheets, spheroids, and colonies of homogenously thickness.

Furthermore, when culturing cells using a substrate in which the ratio (L1/L2) of the width (L1) between convexities of the convex-concave structure and the maximum diameter (L2) of cells to be brought into contact with or held on the substrate is 1 to 300, the number of cells in contact with or held on the bottom surface of a concave portion per cell after inoculating the cells is preferably 1 to 100, more preferably 1 to 50, and even more preferably 1 to 30. Due to this, the cell proliferation property is high due to appropriately and homogenously maintaining the concentration of the cells of the substrate surface after inoculating, and, in the form after culturing, it is additionally possible to prepare cell clusters in a group shape with a homogenously cell concentration, or cell sheets, spheroids, and colonies of homogenously thickness. Here, the number of cells is the number of cells counted by observing the cells using a light-and-shade microscope or the like in consideration of the time until the cells settle on the bottom surface of a concave portion after inoculating, and a number of cells counting cells in contact with or held on a plurality of concave portions is usually handled as an average value divided by the number of concave portions.

The cultured cells prepared using the medical instrument of the present invention are preferably cultured cells maintaining the drug metabolizing system enzyme activity. In other words, the cultured cells are cultured cells which are grown while maintaining substantially the same functions as cells proliferated in vivo and the selection of the type of cell is not limited. Alternatively, it is possible to culture living cells normally except for extremely limited types of cells such as viruses or bacteria with a high possibility of causing gene mutation. Regarding the drug metabolizing system enzyme activity of the cells, the cultured cells are maintained without being at low temperatures or being subjected to a special culture storage operation (for example, storing with an excessive amount of oxygen or the like).

On the other hand, the shape of the cell does not particularly need to be limited; however, examples thereof include sheet-shaped cells, spheroid cells, colony cells, nervous system type cells, and the like. In particular, in the field of regenerative medicine, it is desired to maintain the same drug metabolizing system enzyme activity as living cells as in the present invention and it is important to enable favorable production, as in the cultured cells in the present invention. In addition, the cultured cells of the present invention are expected to maintain the same functions over a long period even in drug development, biological chemicals, and cosmetics and the present invention could be used worldwide.

In addition, forming a substrate using the fluorine-containing cyclic olefin polymer described above makes it possible to improve the formability of the substrate and new medical instrument which has industrial value is realized.

In the present invention, that one surface of a substrate on which cultured cells are in contact or held is formed of the fluorine-containing cyclic olefin polymer described above may include, for example, a case where the substrate is formed by adhering a film formed with a convex-concave structure formed by the fluorine-containing cyclic olefin polymer described above on one surface of the support formed by another material such as plastic or metal, or a case where the entire substrate is formed of the fluorine-containing cyclic olefin polymer by melt molding and a convex-concave structure is formed in a portion with which cells are in contact or held.

In addition, a laminated body in which cultured cells which are produced on the medical instrument of the present invention are covered and laminated with cotton, fabric, non-woven fabric, or the like may be provided. Alternatively, a moisturizer such as glycerine may be impregnated into the laminated body. In other words, with the cultured cells which are produced by the medical instrument of the present invention, medical practices may be performed such as removing a cover of a laminated body, directly attaching this to an affected part as regenerative medicine, and subsequently detaching the film.

In the present invention, it is possible to set the water contact angle on one surface where the convex-concave structure contacting or holding the cultured cells is formed to, for example, 70° to 160°, preferably 75° to 155°, and more preferably 80° to 150°. Due to this, it is possible to more easily float the cells which proliferate without adhesion or attachment to the one surface, for example, using a buffer solution such as phosphate-buffered physiological saline. For this reason, when detaching cells from a substrate, it is possible to more reliably suppress damage to the cells.

It is possible to control the water contact angle on one surface of a substrate by appropriately selecting the material which forms the surface of the substrate or various conditions in the method for producing the substrate. In the present invention, forming the surface of the substrate described above with the substrate formed with a convex-concave structure formed from the fluorine-containing cyclic olefin polymer which contains a structural unit which is represented by General Formula (1) is one of the important elements for setting the water contact angle to be in a desired range.

It is possible to measure the water contact angle on the basis of Japanese Industrial Standard JIS-R 3257 (methods for testing the wettability of a substrate glass surface) using a method of dripping water droplets with a capacity of 4 *µ*l or less, in which the shape of the water droplet is able to be seen as a spherical shape under constant temperature and constant humidity conditions of 25±5°C and 50±10%, onto a substrate surface and measuring the angle of the contact interface between the substrate and the water droplets within one minute after the water droplets come into contact with the substrate surface according to a sessile drop method. In the present invention, for example, it is possible to treat the numeric value within one minute directly after the water droplets come into contact in the method described above as the physical property value in the same manner as the numeric value which is used for the physical property value of various types of plastic material.

Examples of the types of cells which are able to be treated in the medical instrument in the present invention include, in the case of animal cells and regardless of whether floating cells or adherent cells, for example, fibroblasts, mesenchymal stem cells, hematopoietic stem cells, neural stem cells, nerve cells, corneal epithelial cells, mouth mucosa cells, retinal pigment cells, periodontal membrane stem cells, myofibroblasts, cardiac muscle cells, liver cells, pancreatic endocrine cells, dermal keratinocytes, dermal fibroblasts, precursor cells derived from subcutaneous fat, kidney cells, lower hair root sheath cells, nasal mucous membrane epithelial cells, vascular endothelium precursor cells, vascular endothelium cells, vascular smooth muscle cells, osteoblastic cells, cartilage cells, skeletal muscle cells, immortalized cells, cancer cells, keratinocytes, embryonic stem cells (ES cells), EBVphenotypic transformation B cells, induced pluripotent stem cells (iPS cells), and the like and more specific examples thereof include HeLa cells, CHO cells, Cos cells, HL-60 cells, Hs-68 cells, MCF7 cells, Jurkat cells, Vero cells, PC-12 cells, K562 cells, L cells, 293 cells, HepG2 cells, U-937 cells, Caco-2 cells, HT-29 cells, A549 cells, B16 cells, MDCK cells, BALB/3T3 cells, V79 cells, 3T3-L1 cells, NIH/3T3 cells, Raji cells, NSCLC cells, A431 cells, Sf9 cells, SH-SY5Y cells, BHK-21 cells, J774 cells, C2C12 cells, 3T3-Swiss albino cells, MOLT-4 cells, CV-1 cells, F9 cells, MC3T3-E1 cells, HaCaT cells, L5178Y cells, HuH-7 cells, Rat1 cells, Saos-2 cells, TIG cells, CHL cells, WI-38 cells, MRC-5 cells, Hep3B cells, SK-N-SH cells, MIN6 cells, KATO cells, C3H/10T1/2 cells, DT40 cells, PLC/PRF/5 cells, IMR-90 cells, FM3A cells, and the like. The cells may be either primary cells or subcultured cells.

Examples of derivations of these cells include cells of various types of living beings such as humans, dogs, rats, mice, birds, pigs, cows, and insects or tissues which are formed by aggregating the above, organs, microorganisms, viruses, and the like, and more specific examples thereof include human cervical cancer derivations, Chinese hamster ovary derivations, CV-1 cell derivations, human myelocytic leukemia derivations, human breast cancer derivations, human T-cell leukemia derivations, Africa green monkey kidney derivations, human adrenal gland pith pheochromocytomas derivations, human myelocytic leukemia derivations, C3H mouse epithelial tissue derivations, human embryonal kidney derivations, human hepatic cancer derivations, human histiocytic leukemia derivations, human colorectal cancer derivations, human lung cancer derivations, mouse melanoma derivations, dog kidney derivations, Balb/c mouse fetus derivations, Chinese hamster lung derivations, Swiss 3T3 derivations, NIH Swiss mouse fetus derivations, human Burkitt lymphoma derivations, human lung non-small cell cancer derivations, human skin Epidermoid Carcinoid derivations, moth larva ovary derivations, Syrian golden hamster kidney derivations, mouse macrophage derivations, mouse muscular tissue derivations, inferior Swiss mouse fetus derivations, human acute T cell leukemia derivations, mouse EC cell OTT6050 derivations, mouse calvaria derivations, human epidermal keratinocyte derivations, DBA/2 mouse thymic tumor derivations, human stem cell cancer derivations, rat connective tissue derivations, human myelogenous osteosarcoma derivations, human fetal lung derivations, human neuroblast tumor derivations, mouse insulinoma derivations, human gastric cancer derivations, C3H mouse fetus derivations, chicken B cell leukemia derivations, mouse spontaneous mammary tumor derivations, and the like.

### (Fluorine-Containing Cyclic Olefin Polymer Used in Medical Instrument)

The fluorine-containing cyclic olefin polymer used for forming the one surface described above of the medical instrument in which cells are brought into contact with one surface having the convex-concave structure of the substrate in the present invention contains a structural unit represented by General Formula (1) below.

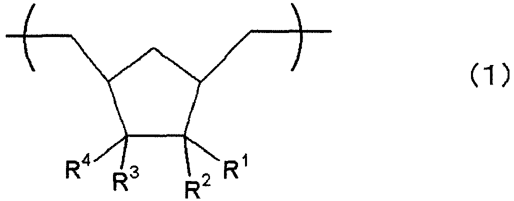

(In Formula (1), at least one of R¹ to R⁴ is fluorine, an alkyl group with 1 to 10 carbon atoms which contains fluorine, an alkoxy group with 1 to 10 carbon atoms which contains fluorine, or an alkoxyalkyl group with 2 to 10 carbon atoms which contains fluorine. R¹ to R⁴ are selected from hydrogen, an alkyl group with 1 to 10 carbon atoms, an alkoxy group with 1 to 10 carbon atoms, or an alkoxyalkyl group with 2 to 10 carbon atoms in a case where R¹ to R⁴ are groups which do not contain fluorine . R¹ to R⁴ may be the same as or different from each other. In addition, R¹ to R⁴ may be bonded to each other to form a cyclic structure.)

Examples of R¹ to R⁴ in General Formula (1) include fluorine; an alkyl group with 1 to 10 carbon atoms which contains fluorine such as an alkyl in which some or all of hydrogen atoms of an alkyl group are substituted with fluorine such as a fluoromethyl group, a difluoromethyl group, a trifluoromethyl group, a trifluoroethyl group, a pentafluoroethyl group, a heptafluoropropyl group, a hexafluoroisopropyl group, a heptafluoroisopropyl group, a hexafluoro-2-methylisopropyl group, a perfluoro-2-methylisopropyl group, an n-perfluorobutyl group, an n-perfluoropentyl group, and a perfluorocyclopentyl group; an alkoxy group with 1 to 10 carbon atoms which contains fluorine such as an alkoxy group in which some or all of hydrogen atoms of an alkoxy group are substituted with fluorine such as a fluoromethoxy group, a difluoromethoxy group, a trifluoromethoxy group, a trifluoroethoxy group, a pentafluoroethoxy group, a heptafluoropropoxy group, a hexafluoroisopropoxy group, a heptafluoroisopropoxy group, a hexafluoro-2-methylisopropoxy group, a perfluoro-2-methylisopropoxy group, an n-perfluorobutoxy group, an n-perfluoropentoxy group, and a perfluorocyclopentoxy group; or an alkoxyalkyl group with 2 to 10 carbon atoms which contains fluorine such as an alkoxyalkyl group in which some or all of hydrogen atoms of an alkoxyalkyl group are substituted with fluorine such as a fluoromethoxy methyl group, a difluoromethoxy methyl group, a trifluoromethoxy methyl group, a trifluoroethoxy methyl group, a pentafluoroethoxy methyl group, a heptafluoropropoxy methyl group, a hexafluoroisopropoxy methyl group, a heptafluoroisopropoxy methyl group, a hexafluoro-2-methylisopropoxy methyl group, a perfluoro-2-methylisopripoxy methyl group, an n-perfluorobutoxy methyl group, an n-perfluoropentoxy methyl group, and a perfluorocyclopentoxy methyl group.

In addition, R¹ to R⁴ may be bonded to each other to form a cyclic structure and may form a ring such as perfluorocycloalkyl and perfluorocycloether via oxygen.

Furthermore, examples of other R¹ to R⁴ which do not contain fluorine include hydrogen; an alkyl group with 1 to 10 carbon atoms such as a methyl group, an ethyl group, a propyl group, an isopropyl group, a 2-methylisopropyl group, an n-butyl group, an n-pentyl group, and a cyclopentyl group; an alkoxy group with 1 to 10 carbon atoms such as a methoxy group, an ethoxy group, a propoxy group, a butoxy group, and a pentoxy group; or an alkoxyalkyl group with 2 to 10 carbon atoms such as a methoxy methyl group, an ethoxy methyl group, a propoxy methyl group, a butoxy methyl group, and a pentoxy methyl group. Preferably, each of R¹ to R⁴ in Formula (1) is a substituent including fluorine or a fluorine atom.

The fluorine-containing cyclic olefin polymer may only have a structural unit which is represented by General Formula (1) or may have another structural unit along with the structural unit which is represented by General Formula (1). In addition, the fluorine-containing cyclic olefin polymer may include two or more types of structural units which are structural units which are represented by General Formula (1) and in which at least one of R¹ to R⁴ is different from each other.

Specific examples of the fluorine-containing cyclic olefin polymer which contains a structural unit which is represented by General Formula (1) in the present invention include poly(1-fluoro-2-trifluoromethyl-3,5-cyclopentylene ethylene), poly(1-fluoro-1-trifluoromethyl-3,5-cyclopentylene ethylene), poly(1-methyl-1-fluoro-2-trifluoromethyl-3,5-cyclopentylene ethylene), poly(1,1-difluoro-2-trifluoromethyl-3,5-cyclopentylene ethylene), poly(1,2-difluoro-2-trifluoromethyl-3,5-cyclopentylene ethylene), poly(1-perfluoroethyl-3,5-cyclopentylene ethylene), poly(1,1-bis(trifluoromethyl)-3,5-cyclopentylene ethylene), poly(1,1,2-trifluoro-2-trifluoromethyl-3,5-cyclopentylene ethylene), poly(1,2-difluoro-1-trifluoroethyl-2-trifluoromethyl-3,5-cyclope ntylene ethylene), poly(1,2-difluoro-1-perfluoro-iso-propyl-2-trifluoromethyl-3,5-c yclopentylene ethylene), poly(1,2-bis(trifluoromethyl)-3,5-cyclopentylene ethylene), poly(1-perfluoropropyl-3,5-cyclopentylene ethylene), poly(1-methyl-2-perfluoropropyl-3,5-cyclopentylene ethylene), poly(1-butyl-2-perfluoropropyl-3,5-cyclopentylene ethylene), poly(1-perfluoro-iso-propyl-3,5-cyclopentylene ethylene), poly(1-methyl-2-perfluoro-iso-propyl-3,5-cyclopentylene ethylene), poly(1,2-difluoro-1,2-bis(trifluoromethyl)-3,5-cyclopentylene ethylene), poly(1,1,2,2,3,3,3a,6a-octafluorocyclopentyl-4,6-cyclopentylene ethylene), poly(1,1,2,2,3,3,4,4,3a,7a-decafluorocyclohexyl-5,7-cyclopentyle ne ethylene), poly(1-perfluorobutyl-3,5-cyclopentylene ethylene), poly(1-perfluoro-iso-butyl-3,5-cyclopentylene ethylene), poly(1-perfluoro-tert-butyl-3,5-cyclopentylene ethylene), poly(1-methyl-2-perfluoro-iso-butyl-3,5-cyclopentylene ethylene), poly(1-butyl-2-perfluoro-iso-butyl-3,5-cyclopentylene ethylene), poly(1,2-difluoro-1-trifluoromethyl-2-perfluoroethyl-3,5-cyclope ntylene ethylene), poly(1-(1-trifluoromethyl-2,2,3,3,4,4,5,5-octafluoro-cyclopentyl )-3,5-cyclopentylene ethylene), poly((1,1,2-trifluoro-2-perfluorobutyl)-3,5-cyclopentylene ethylene), poly(1,2-difluoro-1-trifluoromethyl-2-perfluorobutyl-3,5-cyclope ntylene ethylene), poly(1-fluoro-1-perfluoroethyl-2,2-bis(trifluoromethyl))-3,5-cyc lopentylene ethylene), poly(1,2-difluoro-1-perfluoropropyl-2-trifluoromethyl)-3,5-cyclo pentylene ethylene), poly(1-perfluorohexyl-3,5-cyclopentylene ethylene), poly(1-methyl-2-perfluorohexyl-3,5-cyclopentylene ethylene), poly(1-butyl-2-perfluorohexyl-3,5-cyclopentylene ethylene), poly(1-hexyl-2-perfluorohexyl-3,5-cyclopentylene ethylene), poly(1-octyl-2-perfluorohexyl-3,5-cyclopentylene ethylene), poly(1-perfluoroheptyl-3,5-cyclopentylene ethylene), poly(1-perfluorooctyl-3,5-cyclopentylene ethylene), poly(1-perfluorodecanyl-3,5-cyclopentylene ethylene), poly(1,1,2-trifluoro-perfluoropentyl-3,5-cyclopentylene ethylene), poly(1,2-difluoro-1-trifluoromethyl-2-perfluorobutyl-3,5-cyclope ntylene ethylene), poly(1,1,2-trifluoro-perfluorohexyl-3,5-cyclopentylene ethylene), poly(1,2-difluoro-1-trifluoromethyl-2-perfluoropentyl-3,5-cyclop entylene ethylene), poly(1,2-bis(perfluorobutyl)-3,5-cyclopentylene ethylene), poly(1,2-bis(perfluorohexyl)-3,5-cyclopentylene ethylene), poly(1-methoxy-2-trifluoromethyl-3,5-cyclopentylene ethylene), poly(1-tert-butoxymethyl-2-trifluoromethyl-3,5-cyclopentylene ethylene), poly(1,1,3,3,3a,6a-hexafluorofuranyl-3,5-cyclopentylene ethylene), poly(1-fluoro-2-trifluoromethoxy-3,5-cyclopentylene ethylene), poly(1-fluoro-1-trifluoromethoxy-3,5-cyclopentylene ethylene), poly(1-methyl-1-fluoro-2-trifluoromethoxy-3,5-cyclopentylene ethylene), poly(1,1-difluoro-2-trifluoromethoxy-3,5-cyclopentylene ethylene), poly(1,2-difluoro-2-trifluoromethoxy-3,5-cyclopentylene ethylene), poly(1-perfluoroethoxy-3,5-cyclopentylene ethylene), poly(1,1-bis(trifluoromethoxy)-3,5-cyclopentylene ethylene), poly(1,1,2-trifluoro-2-trifluoromethoxy-3,5-cyclopentylene ethylene), poly(1,2-bis(trifluoromethoxy)-3,5-cyclopentylene ethylene), poly(1-perfluoropropoxy-3,5-cyclopentylene ethylene), poly(1-methyl-2-perfluoropropoxy-3,5-cyclopentylene ethylene), poly(1-butyl-2-perfluoropropoxy-3,5-cyclopentylene ethylene), poly(1-perfluoro-iso-propoxy-3,5-cyclopentylene ethylene), poly(1-methyl-2-perfluoro-iso-propoxy-3,5-cyclopentylene ethylene), poly(1,2-difluoro-1,2-bis(trifluoromethoxy)-3,5-cyclopentylene ethylene), poly(1-perfluorobutoxy-3,5-cyclopentylene ethylene), poly(1-perfluoro-iso-butoxy-3,5-cyclopentylene ethylene), poly(1-perfluoro-tert-butoxy-3,5-cyclopentylene ethylene), poly(1-methyl-2-perfluoro-iso-butoxy-3,5-cyclopentylene ethylene), poly(1-butyl-2-perfluoro-iso-butoxy-3,5-cyclopentylene ethylene), poly(1,2-difluoro-1-trifluoromethoxy-2-perfluoroethoxy-3,5-cyclo pentylene ethylene), poly(1,1,2-trifluoro-2-perfluorobutoxy-3,5-cyclopentylene ethylene), poly(1,2-difluoro-1-trifluoromethoxy-2-perfluorobutoxy-3,5-cyclo pentylene ethylene), poly(1-fluoro-1-perfluoroethoxy-2,2-bis(trifluoromethoxy)-3,5-cy clopentylene ethylene), poly(1,2-difluoro-1-perfluoropropoxy-2-trifluoromethoxy-3,5-cycl opentylene ethylene), poly(1-perfluoroheptoxy-3,5-cyclopentylene ethylene), poly(1-methyl-2-perfluoroheptoxy-3,5-cyclopentylene ethylene), poly(1-butyl-2-perfluoroheptoxy-3,5-cyclopentylene ethylene), poly(1-hexyl-2-perfluoroheptoxy-3,5-cyclopentylene ethylene), poly(1-octyl-2-perfluoroheptoxy-3,5-cyclopentylene ethylene), poly(1-perfluoroheptoxy-3,5-cyclopentylene ethylene), poly(1-perfluorooctoxy-3,5-cyclopentylene ethylene), poly(1-perfluorodesiloxy-3,5-perfluorodecyloxy ethylene), poly(1,1,2-trifluoro-perfluoropentoxy-3,5-cyclopentylene ethylene), poly(1,2-difluoro-1-trifluoromethoxy-2-perfluorobutoxy-3,5-cyclo pentylene ethylene), poly(1,1,2-trifluoro-2-perfluoroheptoxy-3,5-cyclopentylene ethylene), poly(1,2-difluoro-1-trifluoromethoxy-2-perfluoropentyl-3,5-cyclo pentylene ethylene), poly(1,2-bis(perfluorobutoxy)-3,5-cyclopentylene ethylene), poly(1,2-bis(perfluoroheptoxy)-3,5-cyclopentylene ethylene), poly(1-methoxy-2-trifluoromethoxy-3,5-cyclopentylene ethylene), poly(1-tert-butoxymethyl-2-trifluoromethoxy-3,5-cyclopentylene ethylene), poly(1-(2',2',2'-trifluoroethoxy)-3,5-cyclopentylene ethylene), poly(1-(2',2',3',3',3'-pentafluoropropoxy)-3,5-cyclopentylene ethylene), poly(1-methyl-2-(2',2',3',3',3'-pentafluoropropoxy)-3,5-cyclopen tylene ethylene), poly(1-butyl-2-(2',2',3',3',3'-pentafluoropropoxy)-3,5-cyclopent ylene ethylene), poly(1-(1',1',1'-trifluoro-iso-propoxy)-3,5-cyclopentylene ethylene), poly(1-methyl-(1',1',1'-trifluoro-iso-propoxy)-3,5-cyclopentylen e ethylene), poly(1-(2',2',3',3',4',4',4'-heptafluorobutoxy)-3,5-cyclopentyle ne ethylene), poly(1-(1',1',1'-trifluoro-iso-butoxy)-3,5-cyclopentylene ethylene), poly(1-(1',1',1'-trifluoro-iso-butoxy)-3,5-cyclopentylene ethylene), poly(1-methyl-2-(1',1',1'-trifluoro-iso-butoxy)-3,5-cyclopentyle ne ethylene), poly(1-butyl-2-(1',1',1'-trifluoro-iso-butoxy)-3,5-cyclopentylen e ethylene), poly(1,2-difluoro-1-trifluoromethoxy-2-(2',2',2'-trifluoroethoxy )-3,5-cyclopentylene ethylene), poly(1,1,2-trifluoro-2-(2',2',3',3',4',4',4'-heptafluorobutoxy)-3,5-cyclopentylene ethylene), poly(1,2-difluoro-1-trifluoromethoxy-2-(2',2',3',3',4',4',4'-hep tafluorobutoxy)-3,5-cyclopentylene ethylene), poly(1-fluoro-1-(2',2',2'-trifluoroethoxy)-2,2-bis(trifluorometh oxy))-3,5-cyclopentylene ethylene), poly(1,2-difluoro-1-(2',2',3',3',3'-pentafluoropropoxy)-2-triflu oromethoxy-3,5-cyclopentylene ethylene), poly(1-(2',2',3',3',4',4',5',5',6',6',6'-undecafluoroheptoxy)-3, 5-cyclopentylene ethylene), poly(1-methyl-2-(2',2',3',3',4',4',5',5',6',6',6'-undecafluorohe ptoxy)-3,5-cyclopentylene ethylene), poly(1-butyl-2-(2',2',3',3',4',4',5',5',6',6',6'-undecafluorohep toxy)-3,5-cyclopentylene ethylene), poly(1-hexyl-2-(2',2',3',3',4',4',5',5',6',6',6'-undecafluorohep toxy)-3,5-cyclopentylene ethylene), poly(1-octyl-2-(2',2',3',3',4',4',5',5',6',6',6'-undecafluorohep toxy)-3,5-cyclopentylene ethylene), poly(1-(2',2',3',3',4',4',5',5',6',6',7',7',7'-tridecafluorohept oxy)-3,5-cyclopentylene ethylene), poly(1-(2',2',3',3',4',4',5',5',6',6',7',7',8',8',8'-pentadecafl uorooctoxy)-3,5-cyclopentylene ethylene), poly(1-(2',2',3',3',4',4',5',5',6',6',7',7',8',8',9',9',9'-hepta decafluorodecyloxy)-3,5-cyclopentylene ethylene), poly(1,1,2-trifluoro-2-(1',1',1'-trifluoro-iso-propoxy)-3,5-cycl opentylene ethylene), poly(1,2-difluoro-1-trifluoromethoxy-2-(2',2',3',3',4',4',4'-hep tafluorobutoxy)-3,5-cyclopentylene ethylene), poly(1,1,2-trifluoro-(2',2',3',3',4',4',5',5',6',6',6'-undecaflu oroheptoxy)-3,5-cyclopentylene ethylene), poly(1,2-bis(2',2',3',3',4',4',4'-heptafluorobutoxy)-3,5-cyclope ntylene ethylene), poly(1,2-bis(2',2',3',3',4',4',5',5',6',6',6'-undecafluoroheptox y)-3,5-cyclopentylene ethylene), and the like.

The molecular weight of the fluorine-containing cyclic olefin polymer in the present invention is preferably 5,000 to 1,000,000 and more preferably 10,000 to 300,000, for example, in terms of the weight average molecular weight (Mw) as a polystyrene converted value which is measured by a gel permeation chromatography (GPC) method at a sample concentration of 3.0 to 9.0 mg/ml. Setting the weight average molecular weight (Mw) to be the lower limit value described above or more makes it possible to more reliably obtain a mold in a favorable state in which cracks or the like which are caused by external stress such as bending are not generated in a mold formed by a solution casting method or a mold applied to a substrate. In addition, setting the weight average molecular weight (Mw) to be the upper limit value described above or less makes it possible to have a fluidity at which melt molding is easy.

In addition, the molecular weight distribution (Mw/Mn) which is a ratio of the weight average molecular weight (Mw) and the number average molecular weight (Mn) is preferably 1.3 to 5.0, more preferably 1.5 to 4.5, and particularly preferably 1.7 to 4.0. Setting the molecular weight distribution (Mw/Mn) to be the lower limit value described above or more makes it possible to improve the toughness of the films or molds which are produced by various types of molding methods such as a solution casting method or a melt molding method and more effectively suppress the generation of cracks or breaks caused by external stress. On the other hand, setting the molecular weight distribution (Mw/Mn) to be the upper limit value described above or less makes it possible to suppress particularly low molecular weight components such as oligomers from eluting and to more reliably suppress the form of the cell proliferation from being prevented by the water contact angle of the substrate surface formed with a convex-concave structure being changed. Setting the weight average molecular weight (Mw) and the weight molecular distribution (Mw/Mn) to be in the ranges described above makes it possible to obtain particularly favorable medical instrument as used for medical uses or cell culturing and inspection which uses cells.

The glass transition temperature of the fluorine-containing cyclic olefin polymer according to differential scanning calorimetric analysis is preferably 50°C to 300°C, more preferably 80°C to 280°C, and even more preferably 100°C to 250°C. When the glass transition temperature is within the ranges described above, it is possible to perform a heat sterilizing treatment, to maintain the shape in the usage environment and to more favorably obtain medical instrument as a substrate for medical uses or cell culturing and inspection which uses cells, which has excellent fluidity with respect to a heating temperature in melt molding, has good production stability and is also excellent in hue.

The partially fluorinated polymer containing the structural unit represented by General Formula (1) in the present invention is different from the fully fluorinated polymers and has a partially fluorinated polymer structure in which the main chain is a hydrocarbon and the side chain has a fluorine atom, due to which the polarity is large. Due to this, the molded product of the fluorine-containing cyclic olefin polymer of the present invention has a relatively hydrophobic surface property having a water contact angle of 70° or more with respect to the surface properties of glass, polystyrene, or olefin polymers which are commercially available as culture substrates while dissolving well in polar solvents such as ether and ketone which are generally commercially available solvents used at the time of polymer synthesis and also exhibiting excellent solubility in polar compounds such as a photocurable compound. Due to this, it is possible to float cell clusters such as spheroids and colonies in the form of cultured groups, or cell sheets, for example, with a phosphate buffer solution and to easily detach the cells from the substrate.

Examples of the usage form of the resin described above in the present invention include a medical or cell culture bag, a plate, a petri dish, a dish, a flask, a tube, and the like. Here, for example, the cells may be cells from a living organ, blood, or the like.

### (Production Method of Fluorine-Containing Cyclic Olefin Polymer)

Next, description will be given of a method for producing a fluorine-containing cyclic olefin polymer.

By using a molded product formed with a convex-concave structure formed of a fluorine-containing cyclic olefin polymer obtained by the production method described below as a substrate in the medical instrument of the present invention, it is possible to suitably obtain a medical instrument in which the adhesion or attachment of cells is suppressed.

In detail, it is possible to synthesize a fluorine-containing cyclic olefin polymer by chain transfer polymerizing a cyclic olefin monomer which is represented by General Formula (2) below using a ring-opening metathesis polymerization catalyst and hydrogenating the olefin portion of the main chain of the obtained polymer.

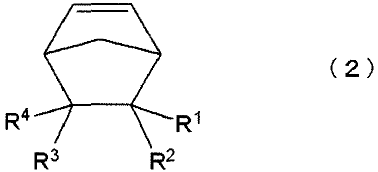

(In Formula (2), R¹ to R⁴ have the same meaning as R¹ to R⁴ in Formula (1). In addition, R¹ to R⁴ may be bonded to each other to form a cyclic structure. Each of R¹ to R⁴ in Formula (2) is preferably a substituent including fluorine or fluorine atom.)

Here, a monomer other than the cyclic olefin monomer which is represented by General Formula (2) may be included within a range which does not inhibit the effects of the present invention.

Here, when synthesizing the fluorine-containing cyclic olefin polymer of the present invention, the monomer represented by General Formula (2) is preferably used in an amount of 90% to 100% by mass of the entire compound contributing to the polymerization, more preferably 95% to 100% by mass, and more preferably from 98% to 100% by mass.

The fluorine-containing cyclic olefin polymer which contains a structural unit which is represented by General Formula (1) of the present invention is a fluorine-containing polymer in which a main chain double bond is hydrogenated (hydrogenation) after carrying out ring-opening metathesis polymerization on the monomer which is represented by General Formula (2). A Schrock catalyst is preferably used for the ring-opening metathesis polymerization but a Grubbs catalyst may also be used and, due to this, polymerization catalytic activity with respect to a polar monomer is increased and it is possible to realize a production method which is excellent for industry. Here, these ring-opening metathesis polymerization catalysts may be used individually or may be used in a combination of two or more types. In addition, it is also possible to use a ring-opening metathesis polymerization catalyst which is formed by a combination of classical organic transition metal complexes, transition metal halogenated compositions, or transition metal oxides and Lewis acid as a co-catalyst.

In addition, it is possible to use an olefin or diene as a chain transfer agent in order to control the molecular weight and the distribution thereof when ring-opening metathesis polymerization is carried out. As the olefins, it is possible to use α-olefins such as ethylene, propylene, 1-butene, 1-pentene, 1-hexene, and 1-octene or fluorine-containing olefins thereof. Furthermore, it is also possible to use silicon-containing olefins such as vinyltrimethylsilane, allyltrimethylsilane, allyltriethylsilane, and allyltriisopropylsilane or fluorine- and silicon-containing olefins of the above as a chain transfer agent. In addition, examples of dienes include non-conjugated dienes such as 1,4-pentadiene, 1,5-hexadiene, and 1,6-heptadiene or fluorine-containing non-conjugated dienes thereof. These olefins, fluorine-containing olefins, or dienes may be each used individually or two or more types may be used together.

In addition, ring-opening metathesis polymerization of monomers may be carried out in the absence or presence of a solvent. Examples of solvents in a case of using solvents include ethers such as tetrahydrofuran, diethyl ether, dibutyl ether, dimethoxyethane, or dioxane, esters such as ethyl acetate, propyl acetate, or butyl acetate, aromatic hydrocarbons such as benzene, toluene, xylene, or ethyl benzene, aliphatic hydrocarbons such as pentane, hexane, or heptane, aliphatic cyclic hydrocarbons such as cyclopentane, cyclohexane, methyl cyclohexane, dimethyl cyclohexane, or decalin, halogenated hydrocarbons such as methylene dichloride, dichloroethane, dichloroethylene, tetrachloroethane, chlorobenzene, or trichlorobenzene, fluorine-containing aromatic hydrocarbons such as fluorobenzene, difluorobenzene, hexafluorobenzene, trifluoromethyl benzene, and metaxylene hexafluoride, fluorine-containing aliphatic hydrocarbons such as perfluorohexane, fluorine-containing aliphatic cyclic hydrocarbons such as perfluorocyclodecalin, or fluorine-containing ethers such as perfluoro-2-butyltetrahydrofuran. These may be used individually or may be used in a combination of two or more types.

In the ring-opening metathesis polymerization of the monomer, although there are differences depending on the reactivity of the monomer and the solubility to a polymerization solvent, the concentration of the monomer with respect to the monomer solution is preferably 5% to 100% by mass and more preferably 10% to 60% by mass. In addition, the reaction temperature is preferably -30°C to 150°C and more preferably 30°C to 100°C. In addition, the reaction time is preferably 10 minutes to 120 hours and more preferably 30 minutes to 48 hours. Furthermore, it is possible to stop the reaction using quenchers such as aldehydes such as butylaldehyde, ketones such as acetones, alcohols such as methanol, or water and to obtain a polymerization solution.

The catalyst for hydrogenating the double bond portion of the main chain of the polymer obtained by ring-opening metathesis polymerization may be either a homogeneous metal complex catalyst or a heterogeneous metal supported catalyst as long as the catalyst is able to carry out hydrogenation. Examples of homogeneous metal complex catalysts include chlorotris(triphenylphosphine)rhodium, dichlorotris(triphenylphosphine)osmium, dichlorohydride bis(triphenylphosphine)iridium, dichlorotris(triphenylphosphine)ruthenium, dichlorotetrakis(triphenylphosphine)ruthenium, chlorohydride carbonyltris(triphenylphosphine)ruthenium, dichlorotris(trimethylphosphine)ruthenium, and the like and also examples of heterogeneous metal supported catalysts include activated carbon supported palladium, alumina supported palladium, activated carbon supported rhodium, alumina supported rhodium, activated carbon supported ruthenium, alumina supported ruthenium, and the like. In the present invention, the hydrogenation catalyst is preferably a heterogeneous metal-supported catalyst which is easily separable, in addition, these may be used individually and use in a combination of two or more types is also possible.

The solvent which is used for hydrogenating is not particularly limited as long as it dissolves a polymer and the solvent itself is not hydrogenated and examples thereof include ethers such as tetrahydrofuran, diethyl ether, dibutyl ether, and dimethoxyethane, esters such as ethyl acetate, propyl acetate, or butyl acetate, aromatic hydrocarbons such as benzene, toluene, xylene, and ethyl benzene, aliphatic hydrocarbons such as pentane, hexane, and heptane, aliphatic cyclic hydrocarbons such as cyclopentane, cyclohexane, methyl cyclohexane, dimethyl cyclohexane, and decalin, halogenated hydrocarbons such as methylene dichloride, chloroform, dichloroethane, dichloroethylene, tetrachloroethane, chlorobenzene, and trichlorobenzene, fluorine-containing aromatic hydrocarbons such as fluorobenzene, difluorobenzene, hexafluorobenzene, trifluoromethyl benzene, and metaxylene hexafluoride, fluorine-containing aliphatic hydrocarbons such as perfluorohexane, fluorine-containing aliphatic cyclic hydrocarbons such as perfluorocyclodecalin, or fluorine-containing ethers such as perfluoro-2-butyltetrahydrofuran, and the like. These may be used individually or may be used in a combination of two or more types.

Regarding the hydrogenating reaction of the olefin portion of the main chain described above, the hydrogen pressure is preferably normal pressure to 10 MPa, more preferably 0.5 to 8 MPa, and particularly preferably 2 to 5 MPa. In addition, the reaction temperature is preferably 0°C to 200°C, more preferably room temperature to 150°C, and particularly preferably 50°C to 100°C. The manner of carrying out the hydrogenating reaction is not particularly limited; however, examples thereof include a method of hydrogenating by dispersing or dissolving a catalyst in a solvent, a method of hydrogenating by filling a catalyst in a column or the like and circulating a polymerization solution as a stationary phase, and the like.

Furthermore, the hydrogenating treatment of the olefin portion of the main chain is not particularly limited, and a hydrogenating treatment may be performed by precipitating a polymerization solution of the polymer before the hydrogenating treatment in a poor solvent, isolating the polymer, and then re-dissolving the resultant in a solvent, or a hydrogenating treatment may be performed by the hydrogenating catalyst described above without isolating the polymer from a polymerization solution.

In addition, the hydrogenating ratio of the olefin portion of the polymer is preferably 80% or more, more preferably 90% to 100%, and even more preferably 95% to 100%. Setting the hydrogenating ratio to the above-described lower limit or more suppresses deterioration due to light absorption and the occurrence of oxidation due to heating during formation in the olefin portion, and it is possible to obtain good adhesion to the substrate. In addition, residual double bonds may interfere with observation of the growing cells by fluorescence microscopy.

In a case of using a preferable solid catalyst such as activated carbon supported palladium and alumina supported palladium in particular after hydrogenating, the method of obtaining the polymer from the polymerization solution is not particularly limited, and examples thereof include a method of obtaining a polymer by a method such as filtration, centrifugal separation, and decantation and discharging a reaction solution in a poor solvent while stirring, a method of precipitating a polymer by a method such as steam stripping which blows steam into a reaction solution, a method of evaporating and removing a solvent from a reaction solution by heating or the like, and the like.

In addition, in a case of carrying out a hydrogenating reaction using a heterogeneous metal supported catalyst, it is also possible to obtain a polymer by the method described above after filtering the synthesized liquid and filtering and separating a metal supported catalyst. Here, a polymer may be obtained by the method described above after precipitating catalyst components with a large particle diameter in a polymerization solution beforehand by a method such as decantation or centrifugal separation, taking the supernatant liquid, and filtering a solution from which the catalyst components are mostly taken out. In particular, precise filtering of the catalyst components is favorable and the aperture of the filtering filter is preferably 10 *µ*m to 0.05 *µ*m, particularly preferably 10 *µ*m to 0.10 *µ*m, and even more preferably 5 *µ*m to 0.10 *µ*m.

### (Method for Producing Medical Instrument Using Fluorine-Containing Cyclic Olefin Polymer or Fluorine-Containing Cyclic Olefin Polymer Composition)

It is possible to use the medical instrument using fluorine-containing cyclic olefin polymer or the fluorine-containing cyclic olefin polymer composition of the present invention for medical use, cell culturing, cell inspection, or the like. Here, description will be given of a method for manufacturing medical instrument. The medical instrument of the present invention may be formed by, for example, bonding a substrate formed of a film or a sheet-like monolayer film formed with a convex-concave structure or a film or a sheet-like monolayer formed with a convex-concave structure formed on another material with the resin of the present invention, another resin, or another material using an adhesive, an adhesive agent, or the like. Furthermore, a convex-concave structure may be formed by forming a convex-concave structure in a roll or a metal mold by a method such as extrusion film molding or injection molding.

Furthermore, specific examples thereof include an imprinting method in which a varnish-like fluorine-containing cyclic olefin polymer or fluorine-containing cyclic olefin polymer composition is applied to a pattern mold by a solution casting method, the solvent is evaporated, and UV irradiation is performed after the solvent evaporation; an imprinting method in which a film is molded in advance and the pattern of the mold is transferred by heat pressing or UV curing, or melt molding forming a convex-concave structure by forming a mold pattern of a convex-concave structure on a roll or a metal mold surface with a method such as extrusion film molding or injection molding or a method of forming a mold pattern by roll to roll UV curing.

The concave portions of the convex-concave structure in the present invention have the width (L1) between convexities, preferably shaped with a pattern of 10 *µ*m to 1,000 *µ*m, more preferably imprinted and shaped with a pattern of 20 *µ*m to 1,000 *µ*m, and even more preferably 50 *µ*m to 1,000 *µ*m and it is possible to stably bring the cells into contact with the bottom surface of A concave portion. In addition, the width of the convex portion of the convex-concave structure is preferably shaped with a pattern of 1 *µ*m to 300 *µ*m, more preferably 3 *µ*m to 300 *µ*m, and even more preferably 5 *µ*m to 300 *µ*m, while the height of the convex portion is preferably shaped with a pattern of 10 *µ*m to 1,000 *µ*m, more preferably 20 *µ*m to 1,000 *µ*m, and even more preferably 50 *µ*m to 1,000 *µ*m. Due to this, it is possible to bring inoculated cells into uniform contact or hold the inoculated cells on the entire surface of the substrate at an appropriate concentration, and it is possible to easily float and detach cell clusters such as colonies and spheroids in a group shape, or cell sheets in the shape after culturing from the substrate with a buffer solution such as phosphate-buffered physiological saline.

The shape of the convex-concave structure is not particularly limited, but the convex-concave structure may be formed in various shapes such as circles, squares, rectangles, regular triangles, and regular hexagons, formed by various methods such as screen printing, embossing, submicron imprinting, or nano-imprinting and the width (L1) between convexities is the maximum length thereof.

In terms of producing the medical instrument of the present invention, the fluorine-containing cyclic olefin polymer or the fluorine-containing cyclic olefin polymer composition containing the structural unit represented by General Formula (1) may be prepared using the following organic solvents. Examples of organic solvents include fluorine-containing aromatic hydrocarbons such as methaxylene hexafluoride, benzotrifluoride, fluorobenzene, difluorobenzene, hexafluorobenzene, trifluoromethylbenzene, and bis(trifluoromethyl)benzene, fluorine-containing aliphatic hydrocarbons such as perfluorohexane and perfluorooctane, fluorine-containing aliphatic cyclic hydrocarbons such as perfluorocyclodecalin, fluorine-containing ethers such as perfluoro-2-butyltetrahydrofuran, halogenated hydrocarbons such as chloroform, chlorobenzene, and trichlorobenzene, ethers such as tetrahydrofuran, dibutyl ether, 1,2-dimethoxyethane, and dioxane, esters such as ethyl acetate, propyl acetate, and butyl acetate, ketones such as methylethyl ketone, methylisobutyl ketone, and cyclohexanone, and the like. It is possible to select from among the above in consideration of the solubility and film-forming properties. In addition, the above may be used individually or may be used in a combination of two or more types. In particular, from the point of view of the film-forming property, a solvent which has a boiling point of 70° or more under atmospheric pressure is preferable. Due to this, it is possible to reliably suppress the evaporation rate from becoming excessively fast, to suppress the occurrence of unevenness on the film surface, and to improve the film thickness accuracy at the time of film formation.

The concentration at which the fluorine-containing cyclic olefin polymer or the fluorine-containing cyclic olefin polymer composition containing the structural unit represented by General Formula (1) is dissolved is preferably 1.0% to 99.0% by mass, more preferably 5.0% to 90.0% by mass, and particularly preferably 10.0% to 80.0% by mass. The concentration may be selected in consideration of the solubility of the polymer, the adaptability to the filtration process, pattern formability, the film-forming property, and the thickness of the film.

Furthermore, other components which are known in the art may be added as necessary in a range which does not inhibit the film characteristics in the present invention. Examples of the other components include modifiers such as an anti-aging agent, a leveling agent, a wettability modifier, a surfactant, and a plasticizing agent, a stabilizer such as an ultraviolet absorbing agent, a preservative, and an anti-microbial agent, a photosensitizer, a silane coupling agent, and the like.

Subsequently, it is possible to pass and filter the varnish of the fluorine-containing cyclic olefin polymer or the fluorine-containing cyclic olefin polymer composition containing the structural unit represented by General Formula (1) through a filter. Due to this, it is possible to greatly reduce the content of polymer insoluble matter, gel, foreign matter, and the like in the varnish, and it is possible to stably form a pattern on the substrate surface as the culturing instrument culturing cells or the inspection instrument performing inspections using cells. In addition, it is possible to uniformly obtain a surface property exhibiting hydrophobicity over the entire surface.

The aperture of the filtering filter is preferably 10 *µ*m to 0.05 *µ*m, particularly preferably 10 *µ*m to 0.1 *µ*m, and even more preferably 5 *µ*m to 0.1 *µ*m. The process of the filtration may be a multi-stage process which sends a polymerization solution from a filter with a large hole diameter to a filter with a small hole diameter or may be a single process which directly sends the varnish to a filter with a small hole diameter. The material of the filter may be formed of an organic material such as Teflon (registered trademark), polypropylene (PP), polyethersulfone (PES), or cellulose or may be formed of an inorganic material such as glass fiber or metal and it is possible to select the material from the varnish characteristics and the process adaptability as long as there is no adverse influence on the cell culturing.

In addition, the method of sending varnish to a filter may be a method which uses a pressure difference or may be a method of sending varnish to a filter by mechanical driving via a screw or the like. Furthermore, the filtration temperature is selected from within a range in consideration of the filter performance, the solution viscosity, and the solubility of a polymer and is preferably -10°C to 200°C, more preferably 0°C to 150°C, and particularly preferably room temperature to 100°C.

Next, an example will be shown of a method for manufacturing a substrate with a convex-concave structure formed on one surface of a film from a varnish in which the fluorine-containing cyclic olefin polymer which contains the structural unit represented by General Formula (1) is dissolved in an organic solvent. In the present invention, a substrate having a convex-concave structure formed by various imprinting methods is prepared.

The mold used for preparing the substrate of the present invention has a fine pattern formed on the surface thereof. Examples of the substrate material of this mold include metal materials such as nickel, iron, stainless steel, germanium, titanium, and silicon, inorganic materials such as glass, quartz, and alumina, resin materials such as polyimide, polyamide, polyester, polycarbonate, polyphenylene ether, polyphenylene sulfide, polyacrylate, polymethacrylate, an epoxy resin, and a silicone resin, carbon material such as diamond and graphite, and the like.

It is possible to obtain the substrate on which the convex-concave structure is formed by bringing the pattern surface of the mold into contact with the varnish described above and transferring the pattern of the mold by evaporating the solvent, or heating and transferring the mold pattern after producing the film, or UV curing and transferring the composition of the present invention applied to the mold pattern.

The method of bringing varnish in which the fluorine-containing cyclic olefin polymer which contains the structural unit represented by General Formula (1) of the present invention is dissolved in an organic solvent into contact with the mold is not particularly limited and may be either a method of applying a polymerization solution (varnish) on a fine pattern surface of a mold by a method such as table coating, spin coating, die coating, spray coating, bar coating, or roll coating or a method of applying a polymerization solution on a substrate of a metal material such as stainless steel or silicon, an inorganic material such as glass and quartz, a resin material such as polyimide, polyamide, polyester, polycarbonate, polyphenylene ether, polyphenylene sulfide, polyacrylate, polymethacrylate, an epoxy resin, and a silicone resin, or the like by a method such as table coating, spin coating, die coating, spray coating, bar coating, or roll coating and covering the fine pattern surface of the mold so as to be brought into contact therewith.

In detail, examples thereof include a method (A) which includes a step of applying a solution (varnish) which is formed by a fluorine-containing cyclic olefin polymer and an organic solvent on a mold surface which has a fine pattern and a step of evaporating the solvent from the solution, and a method (B) which includes a step of applying a solution (varnish) which is formed by a fluorine-containing cyclic olefin polymer and an organic solvent on a support (a substrate), a step of pressing the upper surface of the applied layer with the mold surface in which the fine pattern is formed, and a step of evaporating the solvent from the applied layer, and the like. Here, in the method (B), it is also possible to carry out pressing with the mold after evaporating the solvent from the applied layer.

It is possible to carry out the method with the temperature for evaporating the solvent from a transfer body and drying in the range of generally 10°C to 300°C, preferably 50°C to 200°C, the pressure generally in the range of 133 Pa to atmospheric pressure, and moreover with a drying time which is generally 10 minutes to 120 hours, preferably in the range of 30 minutes to 48 hours. In addition, the drying temperature, pressure, and time may be changed in stages with individual settings.

In the present invention, after the solvent is evaporated to form a transfer body on the mold, the transfer body is peeled off to obtain a substrate. The separation of the transfer body is preferably performed at a temperature of a glass transition temperature or less and moreover, separation is more preferably carried out at a temperature of (a glass transition temperature -20°C) or less. Due to this, it is possible to hold a pattern shape which is formed on the transfer body with high precision and easily carry out separation. Regarding the separation method, separation is possible by releasing from the mold through separation or by using surface tension after bringing the mold into contact with the transfer body by, for example, a method such as dipping or spraying with a medium such as water. In addition, the substrate may be separated from the support by adhering a resin material or an inorganic material such as glass to the transfer body rear surface.

In addition, it is also possible to obtain the substrate of the present invention on which the convex-concave structure is formed by bringing a film including a fluorine-containing cyclic olefin polymer or a fluorine-containing cyclic olefin polymer composition containing a structural unit represented by General Formula (1) into contact with a pattern surface of a mold and pressing the film thereon and transferring the pattern of the mold by heating or UV curing.

For example, a method of crimping the mold which is heated to a glass transition temperature or higher to a film, a method of heating a film to a glass transition temperature or higher and crimping a mold, or a method of heating a film and a mold to a glass transition temperature or higher and crimping the mold is preferable, the heating temperature is in the range of the glass transition temperature to (the glass transition temperature +100°C), preferably (the glass transition temperature +5°C) to (the glass transition temperature +50°C), moreover, the crimping pressure is generally 1 MPa to 100 MPa, preferably in the range of 1 MPa to 60 MPa. Due to this, it is possible to form the pattern shape which is formed on the transfer body with high precision.

The separation of the transfer body which is formed on the mold by crimping is preferably performed at the temperature of the glass transition temperature or less and moreover, the separation is more preferably carried out at a temperature of (the glass transition temperature -20°C) or lower. Due to this, it is possible to hold the pattern shape which is formed on the transfer body with high precision and easily carry out the separation. Regarding the separation method, separation is possible by separation from the mold or by using surface tension after bringing the mold into contact with the transfer body by, for example, a method such as dipping or spraying with a medium such as water. In addition, the substrate may be separated from the support by adhering a resin material or an inorganic material such as glass to the transfer body rear surface.

Furthermore, examples thereof include a method of producing a film which is a substrate in which a convex-concave structure is formed by a melt molding method. Examples of a method for producing a film by a melt molding method include a method of making the fluorine-containing cyclic olefin polymer exemplified in the description above into a film via a T-die using a melt kneading apparatus. In melt-extruded film production using a T-die, for example, it is possible to carry out processing into a film in which a convex-concave structure is formed by inserting a cyclic olefin polymer in which an additive agent is blended as necessary in an extruding apparatus, melting and kneading at a temperature which is preferably 50°C to 200°C higher than the glass transition temperature, more preferably 80°C to 150°C higher than the glass transition temperature, extruding from the T-die, and pressing a mold in a roll shape which has a fine pattern on the surface, which is heated to the glass transition temperature of the polymer or higher, onto the film while carrying out feeding using a cooling roll. The heating temperature of the roll which has a fine pattern on the surface at this time uses the same range as the heating temperature at the time of forming the convex-concave structure by heating and crimping the film and the mold described above. In addition, an additive agent such as an ultraviolet absorbing agent, an anti-oxidant, a flame retardant, an anti-static agent, and a coloring agent may be added within a range which does not inhibit the effects of the present invention.

In the present invention, for example, the film thickness of the film substrate produced using the fluorine-containing cyclic olefin polymer or the fluorine-containing cyclic olefin polymer composition is preferably 1 to 10,000 *µ*m, more preferably 5 to 5,000 *µ*m, and particularly preferably 10 to 2,000 *µ*m. These are favorable ranges from the point of view of producing medical instrument which is used for cell culturing such as, for example, a culture bag, a culture plate, and a culture petri dish. The film thickness of the substrate referred to here is the film thickness of the film forming the substrate, and it is possible to appropriately set the film thickness in accordance with the process of manufacturing each tool and the use of the appliance. No particular limitation is imposed on the injection molded substrate.

It is possible to use the films, sheets, and injection molded articles formed with a convex-concave structure produced by a solution casting method, a heat pressing method, or a melt molding method using the fluorine-containing cyclic olefin polymer or the fluorine-containing cyclic olefin polymer composition of the present invention to manufacture medical instrument in the form of a bag, a tube, a molded article, or the like using a heat sealing method, a sealing method using an adhesive, melt extrusion, melt molding, or the like. In addition, it is possible to manufacture medical instrument in the form combining a film, sheet, or melt-molded product obtained by the present method with, for example, a petri dish, multi-well plate, flask, or the like made of other materials such as polystyrene, polyethylene, or metal.

The fluorine-containing cyclic olefin polymer composition of the present invention includes the fluorine-containing cyclic olefin polymer containing the structural unit represented by General Formula (1) exemplified above, a photocurable compound, and a photo-curing initiator. According to the present invention, using such a fluorine-containing cyclic olefin polymer composition makes it possible to form one surface of the substrate having the convex-concave structure in the medical instrument.

Due to this, it is possible to modify the properties or the like of various types of culturing instrument such as a cell culture bag or a plate while exhibiting a strong close contact property with various types of members and while precisely transferring the shape and size of the mold and to provide medical instrument which is able to allow cell proliferation while suppressing the adhesion and attachment of the cells.

Furthermore, the fluorine-containing cyclic olefin polymer composition of the present invention may be obtained, for example, by mixing a fluorine-containing cyclic olefin polymer at an arbitrary concentration with a photocurable compound and a photo-curing initiator.

The organic solvent which is used when preparing the fluorine-containing cyclic olefin polymer composition is not particularly limited and examples thereof include fluorine-containing aromatic hydrocarbons such as metaxylene hexafluoride, benzotrifluoride, fluorobenzene, difluorobenzene, hexafluorobenzene, trifluoromethylbenzene, and bis(trifluoromethyl)benzene, fluorine-containing aliphatic hydrocarbons such as perfluorohexane and perfluorooctane, fluorine-containing aliphatic cyclic hydrocarbons such as perfluorocyclodecalin, fluorine-containing ethers such as perfluoro-2-butyl tetrahydrofuran, halogenated hydrocarbons such as chloroform, chlorobenzene, and trichlorobenzene, ethers such as tetrahydrofuran, dibutyl ether, 1,2-dimethoxyethane, dioxane, propylene glycol monomethyl ether, dipropylene glycol monomethyl ether, and propylene glycol monomethyl ether acetate, esters such as ethyl acetate, propyl acetate, and butyl acetate, ketones such as methylethyl ketone, methylisobutyl ketone, and cyclohexanone, alcohols such as methanol, ethanol, isopropyl alcohol, 2-methoxy ethanol, and 3-methoxy propanol, and the like. In particular, it is possible to use the photocurable compound itself as a preparation solvent.

Among these, in particular, from the point of view of film formability, a solvent having a boiling point of 70°C or more under atmospheric pressure is particularly preferable. Due to this, it is possible to reliably suppress the evaporation speed from being excessively fast. For this reason, it is possible to reliably suppress deterioration of the film thickness precision or unevenness on the film surface, caused by the solvent starting to partially dry during application.

Here, it is also possible to add components known in the art other than the fluorine-containing cyclic olefin polymer, the photocurable compound, and the photo-curing initiator to the fluorine-containing cyclic olefin polymer composition as necessary. Examples of the components include modifiers such as anti-aging agents, leveling agents, wettability modifiers, surfactants, and plasticizing agents, stabilizers such as ultraviolet absorbing agents, preservatives, anti-microbial agents, photosensitizers, silane coupling agents, and the like.

The photocurable compound in the fluorine-containing cyclic olefin polymer composition of the present invention preferably has a mass ratio (fluorine-containing cyclic olefin polymer/photocurable compound) of the fluorine-containing cyclic olefin polymer and the photocurable compound of 99.9/0.1 to 50/50, more preferably 99.9/0.1 to 55/45, and even more preferably 99.9/0.1 to 60/40. Examples of the photocurable compound include a cationically polymerizable ring-opening polymerizable compound, a compound having a reactive double bond group, and the like. A cationically polymerizable ring-opening polymerizable compound is preferably selected from the point of view of suppression of substrate deformation which comes along with volume contraction after being cured when coated and used, or of the compatibility with the fluorine-containing cyclic olefin polymer.

The cationically polymerizable ring-opening polymerizable compound and the compound having a reactive double bond group may have one reactive group in one molecule or may have a plurality thereof. In addition, compounds with different numbers of reactive groups may be blended in the photocurable compound at an arbitrary ratio and used. Furthermore, a compound in which a compound which has a reactive double bond group and a cationically polymerizable ring-opening polymerizable compound are blended at an arbitrary ratio may be used as the photocurable compound. Due to this, it is possible to obtain firm close contact with a member which is formed by another material with dimensional accuracy while transferring the pattern of the mold with the fluorine-containing cyclic olefin polymer composition of the present invention with good dimensional accuracy. In addition, it is possible to favorably obtain medical instrument as a substrate for cell culturing or inspecting cells which is able to realize the effects of the present invention.

Examples of the cationically polymerizable ring-opening polymerizable compounds in the photocurable compounds include epoxy compounds such as cyclohexene epoxide, dicyclopentadiene oxide, limonene dioxide, 4-vinylcyclohexene dioxide, 3,4-epoxycyclohexylmethyl-3',4'-epoxycyclohexane carboxylate, di(3,4-epoxycyclohexyl)adipate, (3,4-epoxycyclohexyl)methyl alcohol, (3,4-epoxy-6-methylcyclohexyl)methyl-3,4-epoxy-6-methylcyclohexa ne carboxylate, ethylene-1,2-di(3,4-epoxycyclohexane carboxylic acid) ester, (3,4-epoxycyclohexyl)ethyl trimethoxy silane, phenyl glycidyl ether, dicyclohexyl-3,3'-diepoxide, 1,7-octadiene diepoxide, bisphenol A type epoxy resin, halogenated bisphenol A type epoxy resin, bisphenol F type epoxy resin, o-, m-, p-cresol novolac type epoxy resins, phenol novolac type epoxy resin, polyglycidyl ethers of polyhydric alcohols, alicyclic epoxy resins such as 3,4-epoxy-cyclohexenyl methyl-3',4'-epoxy cyclohexene carboxylate, or epoxy compounds such as glycidyl ether of hydrogenated bisphenol A.

Furthermore, examples include 3-methyl-3-(butoxymethyl)oxetane, 3-methyl-3-(pentyloxymethyl)oxetane, 3-methyl-3-(hexyloxymethyl)oxetane, 3-methyl-3-(2-ethylhexyloxymethyl)oxetane, 3-methyl-3-(octyloxycarboxymethyl)oxetane, 3-methyl-3-(decanyloxymethyl)oxetane, 3-methyl-3-(dodecanyloxymethyl)oxetane, 3-methyl-3-(phenoxymethyl)oxetane, 3-ethyl-3-(butoxymethyl)oxetane, 3-ethyl-3-(pentyloxymethyl)oxetane, 3-ethyl-3-(hexyloxymethyl)oxetane, 3-ethyl-3-(2-ethylhexyloxymethyl)oxetane, 3-ethyl-3-(octyloxymethyl)oxetane, 3-ethyl-3-(decanyloxymethyl)oxetane, 3-ethyl-3-(dodecanyloxymethyl)oxetane, 3-(cyclohexyloxymethyl)oxetane, 3-methyl-3-(cyclohexyloxymethyl)oxetane, 3-ethyl-3-(cyclohexyloxymethyl)oxetane, 3-ethyl-3-(phenoxymethyl)oxetane, 3,3-dimethyl oxetane, 3-hydroxymethyl oxetane, 3-methyl-3-hydroxymethyl oxetane, 3-ethyl-3-hydroxymethyl oxetane, 3-ethyl-3-phenoxymethyl oxetane, 3-n-propyl-3-hydroxymethyl oxetane, 3-isopropyl-3-hydroxymethyl oxetane, 3-n-butyl-3-hydroxymethyl oxetane, 3-isobutyl-3-hydroxy methyl oxetane, 3-sec-butyl-3-hydroxymethyl oxetane, 3-tert-butyl-3-hydroxymethyl oxetane, 3-ethyl-3-(2-ethylhexyl)oxetane, or the like.

As a compound which has two or more oxetanyl groups, other examples include oxetane compounds such as bis(3-ethyl-3-oxetanylmethyl)ether(3-ethyl-3 {[(3-ethyloxetan-3-yl) methoxy] methyl} oxetane), 1,2-bis[(3-ethyl-3-oxetanylmethoxy)]ethane, 1,3-bis[(3-ethyl-3-oxetanylmethoxy)]propane, 1,3-bis[(3-ethyl-3-oxetanylmethoxy)]-2,2-dimethyl-propane, 1,4-bis(3-ethyl-3-oxetanylmethoxy)butane, 1,6-bis(3-ethyl-3-oxetanylmethoxy)hexane, 1,4-bis[(3-methyl-3-oxetanyl)methoxy]benzene, 1,3-bis[(3-methyl-3-oxetanyl)methoxy]benzene, 1,4-bis{[(3-methyl-3-oxetanyl)methoxy]methyl}benzene, 1,4-bis{[(3-methyl-3-oxetanyl)methoxy]methyl}cyclohexane, 4,4'-bis{[(3-methyl-3-oxetanyl)methoxy]methyl}biphenyl, 4,4'-bis{[(3-methyl-3-oxetanyl)methoxy]methyl}bicyclohexane, 2,3-bis[(3-methyl-3-oxetanyl)methoxy]bicyclo[2.2.1]heptane, 2,5-bis[(3-methyl-3-oxetanyl)methoxy]bicyclo[2.2.1]heptane, 2,6-bis[(3-methyl-3-oxetanyl)methoxy]bicyclo[2.2.1]heptane, 1,4-bis[(3-ethyl-3-oxetanyl)methoxy]benzene, 1,3-bis[(3-ethyl-3-oxetanyl)methoxy]benzene, 1,4-bis{[(3-ethyl-3-oxetanyl)methoxy]methyl}benzene, 1,4-bis{[(3-ethyl-3-oxetanyl)methoxy]methyl}cyclohexane, 4,4'-bis{[(3-ethyl-3-oxetanyl)methoxy]methyl}biphenyl, 4,4'-bis{[(3-ethyl-3-oxetanyl)methoxy]methyl}bicyclohexane, 2,3-bis[(3-ethyl-3-oxetanyl)methoxy]bicyclo[2.2.1]heptane, 2,5-bis[(3-ethyl-3-oxetanyl)methoxy]bicyclo[2.2.1]heptane, and 2,6-bis[(3-ethyl-3-oxetanyl)methoxy]bicyclo[2.2.1]heptane. The above may be used individually or may be used in a combination of two or more types.

In addition, examples of the compound which has a reactive double bond group in the photocurable compound include olefins such as fluorodiene (CF₂=CFOCF₂CF₂CF=CF₂, CF₂=CFOCF₂CF(CF₃)CF=CF₂, CF₂=CFCF₂C(OH) (CF₃)CH₂CH=CH₂, CF₂=CFCF₂C(OH) (CF₃)CH=CH₂, CF₂=CFCF₂C(CF₃)(OCH₂OCH₃)CH₂CH=CH₂, CF₂=CFCH₂C(C(CF₃)₂OH) (CF₃)CH₂CH=CH₂); cyclic olef ins such as norbornene and norbornadiene; alkyl vinyl ethers such as cyclohexylmethyl vinyl ether, isobutyl vinyl ether, cyclohexyl vinyl ether, and ethyl vinyl ether; vinyl esters such as vinyl acetate; (meth) acrylic acid such as (meth) acrylic acid, phenoxyethyl acrylate, benzyl acrylate, stearyl acrylate, lauryl acrylate, 2-ethylhexyl acrylate, allyl acrylate, 1,3-butanediol diacrylate, 1,4-butanediol diacrylate, 1,6-hexane diol diacrylate, trimethylol propane triacrylate, pentaerythritol triacrylate, dipentaerythritol hexaacrylate, ethoxy ethyl acrylate, methoxyethyl acrylate, glycidyl acrylate, tetrahydrofurfuryl acrylate, diethylene glycol diacrylate, neopentyl glycol diacrylate, polyoxyethylene glycol diacrylate, tripropylene glycol diacrylate, 2-hydroxyethyl acrylate, 2-hydroxypropyl acrylate, 4-hydroxybutyl vinyl ether, N,N-diethylaminoethyl acrylate, N,N-dimethylaminoethyl acrylate, N-vinylpyrrolidone, and dimethyl aminoethyl methacrylate, and derivatives thereof, or fluorine-containing acrylates thereof, derivatives thereof, or the like. The above may be used individually or may be used in a combination of two or more types.

Examples of the photo-curing initiator for curing the monomer described above of the present invention include cationic photo-curing initiators which generate cations when irradiated with light, photoradical initiators which generate radicals when irradiated with light, and the like. The usage amount of the photo-curing initiator is preferably 0.05 parts by mass or more with respect to 100 parts by mass of the photocurable compound, and more preferably from 0.1 to 10 parts by mass.

The cationic photo-curing initiator which generates cations when irradiated with light of the photo-curing initiator is not particularly limited as long as it is a compound which initiates cationic polymerization of the cationically polymerizable ring-opening compounds polymerizable when irradiated with light; however, for example, compounds which undergo a photoreaction and release Lewis acid such as onium salts with anions which form a pair with onium cations are preferable.

Specific examples of the onium cations include diphenyliodonium, 4-methoxy-diphenyliodonium, bis(4-methylphenyl)iodonium, bis(4-tert-butylphenyl)iodonium, bis(dodecyl phenyl)iodonium, triphenylsulfonium, diphenyl-4-thio phenoxyphenyl sulfonium, bis[4-(diphenyl sulfonio)-phenyl]sulfide, bis[4-(di(4-(2-hydroxyethyl)phenyl)sulfonyl)-phenyl]sulfide, η⁵-2,4-(cyclo pentadienyl)[1,2,3,4,5,6-η-(methyl ethyl)benzene]-iron (1+), and the like. In addition, other than the onium cations, examples include perchlorate ions, trifluoromethanesulfonate ions, toluene sulfonate ions, trinitrotoluene sulfonate ions, and the like. In addition, these cationic photo-curing initiators may be used alone or may be used in a combination of two or more types.

On the other hand, specific examples of anions include tetrafluoroborate, hexafluorophosphate, hexafluoroantimonate, hexafluoroarsenate, hexachloroantimonate, tetra(fluorophenyl)borate, tetra(difluorophenyl)borate, tetra(trifluorophenyl)borate, tetra(tetrafluorophenyl)borate, tetra(pentafluorophenyl)borate, tetra(perfluorophenyl)borate, tetra(trifluoromethylphenyl)borate, tetra[di(trifluoromethyl)phenyl)]borate, and the like. In addition, these cationic photo-curing initiators may be used alone or may be used in a combination of two or more types.

Further specific examples of preferably used cationic photo-curing initiators include IRGACURE 250 (produced by BASF), IRGACURE 290 (produced by BASF), IRGACURE 784 (produced by BASF), ESACURE 1064 (produced by Orchid Bell Tea Co., Ltd.), WPI-124 (produced by Wako Pure Chemical Industries Ltd.), CYRAURE UVI6990 (produced by Union Carbide Japan Corporation), CPI-100P (San-Apro Ltd.), Photo Initiator 2074 (produced by Solvay Japan Co., Ltd.), ADEKAOPTOMER SP-172 (Adeka Corporation), ADEKAOPTOMER SP-170 (Adeka Corporation), ADEKAOPTOMER SP-152 (Adeka Corporation), ADEKAOPTOMER SP-150 (Adeka Corporation), and the like. In addition, these cationic photo-curing initiators may be used alone or may be used in a combination of two or more types.

In addition, examples of photoradical initiators generating radicals when irradiated with light in the photo-curing initiator include acetophenones such as acetophenone, p-tert-butyl trichloroacetophenone, chloroacetophenone, 2,2-diethoxyacetophenone, hydroxyacetophenone, 2,2-dimethoxy-2'-phenyl acetophenone, 2-aminoacetophenone, and dialkylamino acetophenone; benzoins such as benzoin, benzoin methyl ether, benzoin ethyl ether, benzoin isopropyl ether, benzoin isobutyl ether, 1-hydroxy cyclohexyl phenyl ketone, 2-hydroxy-2-methyl-1-phenyl-2-methylpropan-1-one, 1-(4-isopropylphenyl)-2-hydroxy-2-methylpropan-1-one; benzophenones such as benzophenone, benzoyl benzoic acid, methyl benzoyl benzoic acid, methyl-o-benzoyl benzoate, 4-phenyl benzophenone, hydroxybenzophenone, hydroxypropyl benzophenone, acrylic benzophenone, and 4,4'-bis(dimethylamino) benzophenone; thioxanthones such as thioxanthone, 2-chloro-thioxanthone, 2-methyl-thioxanthone, diethylthioxanthone, and dimethyl thioxanthone; fluorine-based peroxides such as perfluoro (tert-butyl peroxide), perfluoro benzoyl peroxide; as well as α-acyl oxime ester, benzyl-(o-ethoxycarbonyl)-α-monoxime, acyl phosphine oxide, glyoxy ester, 3-ketocoumarin, 2-ethyl anthraquinone, camphor quinone, tetramethyl thiuram sulfide, azobisisobutyronitrile, benzoyl peroxide, dialkyl peroxide, tert-butyl peroxypivalate, and the like.

Specific examples of more preferably used photoradical initiators include IRGACURE 651 (produced by BASF), IRGACURE 184 (produced by BASF), Darocure 1173 (produced by BASF), benzophenone, 4-phenyl benzophenone, IRGACURE 500 (produced by BASF), IRGACURE 2959 (produced by BASF), IRGACURE 127 (produced by BASF), IRGACURE 907 (produced by BASF), IRGACURE 369 (produced by BASF), IRGACURE 1300 (produced by BASF), IRGACURE 819 (produced by BASF), IRGACURE 1800 (produced by BASF), DAROCURE TPO (produced by BASF), DAROCURE 4265 (produced by BASF), IRGACURE OXE01 (produced by BASF), IRGACURE OXE02 (produced by BASF), ESACURE KT55 (produced by Orchid Bell Tea Co., Ltd.), ESACURE KIP150 (produced by Orchid Bell Tea Co. , Ltd.), ESACURE KIP100F (produced by Orchid Bell Tea Co., Ltd.), ESACURE KT37 (produced by Orchid Bell Tea Co., Ltd.), ESACURE KTO46 (produced by Orchid Bell Tea Co., Ltd.), ESACURE 1001M (produced by Orchid Bell Tea Co. , Ltd.), ESACURE KIP/EM (produced by Orchid Bell Tea Co., Ltd.), ESACURE DP250 (produced by Orchid Bell Tea Co., Ltd.), ESACURE KB1 (produced by Orchid Bell Tea Co., Ltd.), 2,4-diethyl thioxanthone, and the like. Among the above, examples of more preferably used photoradical polymerization initiators include IRGACURE 184 (produced by BASF), DAROCURE 1173 (produced by BASF), IRGACURE 500 (produced by BASF), IRGACURE 819 (produced by BASF), DAROCURE TPO (produced by BASF), ESACURE KIP100F (produced by Orchid Bell Tea Co. , Ltd.), ESACURE KT37 (produced by Orchid Bell Tea Co., Ltd.), and ESACURE KTO46 (produced by Orchid Bell Tea Co. , Ltd.). In addition, these photoradical initiators may be used alone or may be used in a combination of two or more types.

Furthermore, other known components may be added as a third component as necessary, for example, modifiers such as anti-aging agents, leveling agents, wettability improving agents, surfactants, plasticizers, stabilizers such as ultraviolet absorbers, preservatives, anti-microbial agents, photosensitizers, silane coupling agents, and the like.

In addition, regarding the fluorine-containing cyclic olefin polymer composition of the present invention, it is possible for the photocurable compound to form a three-dimensional mesh structure in a form after being cured and it is possible to modify the surface hardness to be harder. For this reason, it is possible to improve the scratch property in a case of implementation in medical instrument or the like and convenient use is possible when used as a substrate for cell culturing or inspection of cells.

It is possible for the photocurable composition of the present invention to be filtered and refined in the same manner as the fluorine-containing cyclic olefin polymer varnish represented by General Formula (1) of the present invention described above.

Next, using a varnish formed of a photocurable composition including a fluorine-containing cyclic olefin polymer containing a structural unit represented by General Formula (1), a photocurable compound, and a photo-curing initiator, the method of forming the convex-concave structure of the substrate is able to obtain the convex-concave structure by transferring the pattern of the mold by irradiation with UV and curing after undergoing the same application step as the fluorine-containing cyclic olefin polymer varnish described above.

The UV irradiation step of UV irradiation and curing may also serve to carry out sterilization, but the irradiation light is not particularly limited as long as it is possible to impart energy which causes a radical reaction or ionic reaction by the photo-curing initiator (C) being irradiated with light. As the light source, it is possible to use rays with a wavelength of 400 nm or less, for example, a low-pressure mercury lamp, a medium pressure mercury lamp, a high-pressure mercury lamp, an ultra-high pressure mercury lamp, a chemical lamp, a black light lamp, a microwave excitation mercury lamp, a metal halide lamp, an i line, a G line, a KrF excimer laser light, and an ArF excimer laser light.

The irradiation strength to the film described above which is formed by the fluorine-containing cyclic olefin polymer composition is controlled according to each target product and is not particularly limited. For example, the light irradiation strength of the light wavelength region (although it is different according to the photo-curing initiator, for example, light of 300 to 420 nm is used) which is effective for activation of the photo-curing initiator is preferably 0.1 to 100 mW/cm². Setting the irradiation strength to be 0.1 mW/cm² or more makes it possible to reliably suppress the reaction time from being excessively long. On the other hand, setting the irradiation strength to be 100 mW/cm² or less makes it possible to reliably suppress decreases in the aggregability of the obtained cured matter, yellowing, or deterioration of the support caused by heat radiated from the lamp and heating the composition at the time of the polymerization of the composition.

The irradiation time of the light is controlled for each desired product and is not particularly limited; however, it is possible to set the accumulated light quantity which is represented as a product of the light irradiation strength in the light wavelength region and the light irradiation time to be, for example, 3 to 1,000 mJ/cm². 5 to 500 mJ/cm² is more preferable and 10 to 300 mJ/cm² is particularly preferable. Setting the accumulated light quantity to be the lower limit value described or more makes it possible to make the generation of active species from the photo-polymerization initiator sufficient and to improve the characteristics of the obtained cured matter. On the other hand, it is possible to contribute to an improvement in the productivity by setting the accumulated light quantity to be the upper limit value described above or less. In addition, heating is also preferably used in order to promote the polymerization reaction in some cases. In addition, the temperature in a case of curing the curable resin by irradiation with light is generally preferably 0°C to 150°C and more preferably 0°C to 60°C.

In a case of obtaining a substrate which is formed by a film or a single layer film in sheet form, for example, it is possible to produce a substrate by separating the film from the mold. The separation of the film from a support may be performed, for example, by adhering a commercially available tape on an end portion of the film and separating by applying pressure thereto or may be performed by separating the film using the difference in surface tension between the support surface and the contact surface of the film by bringing a liquid such as water and a solvent into contact with the contact interface of the film and the support.

In the case of obtaining a substrate on which a coating film having a convex-concave structure formed thereon is formed on a support such as plastic or metal, for example, the steps up to the step of drying the applied film in the above steps are carried out to coat a fluorine-containing cyclic olefin polymer composition on the support. Subsequently, it is possible to produce a substrate in which the applied film on which a convex-concave structure which is formed by the fluorine-containing cyclic olefin polymer composition of the present invention is formed on the support by bringing the pattern surface of the mold into contact with the applied surface of the fluorine-containing cyclic olefin polymer composition, crimping as necessary, and carrying out UV irradiation and separation. In this case, the support is particularly preferably selected from an organic material such as polyethylene terephthalate (PET) and an acryl resin or an inorganic material such as glass, silicon, and aluminum.

The pressure used when bringing the pattern surface of the mold into contact with the applied surface of the fluorine-containing cyclic olefin polymer composition is in the range of 0.01 to 5 MPa, preferably 0.05 to 4 MPa, and more preferably 0.1 to 3 MPa and UV irradiation may be carried out while crimping or UV irradiation may be carried out after crimping using a crimping apparatus such as a laminator. Due to this, regardless of the shape or size of the pattern, it is possible to produce a substrate, in which the pattern of the mold is transferred with high precision.

The film thickness of the cured film which is obtained by film-forming a fluorine-containing cyclic olefin polymer composition of the present invention on a support formed of plastic or metal, heating, and curing by irradiation with light after crimping as necessary is not particularly limited; however, 1 *µ*m to 10,000 *µ*m is preferable, 5 *µ*m to 5, 000 *µ*m is more preferable, and 10 *µ*m to 2, 000 *µ*m is even more preferable. When in these ranges, it is possible to obtain an independent single layer film or applied film on the support. In addition, for the fluorine-containing cyclic olefin polymer composition of the present invention, it is also possible to make the volume contraction small during photo-curing and reliably suppress deformation of the substrate while transferring the pattern with high precision. In addition, these are favorable ranges, for example, from the point of view of producing medical instrument which is used for cell culturing such as a culture bag, a culture plate, and a culture petri dish. Furthermore, it is possible to appropriately set the thickness of the film to match the process of producing the instrument.

In the substrate having the convex-concave structure produced by the method described above from the fluorine-containing cyclic olefin polymer or the fluorine-containing cyclic olefin polymer composition of the present invention, the convex-concave structure may be changed into a shape having an inclined structure from the apex of the convex portion toward the bottom surface of a concave portion by heating, melting, and flowing the substrate after peeling from the mold. Due to this, it is possible to culture the inoculated cells in a state in which the cells are effectively assembled on the bottom surface of a concave portion, and it is possible for the cultured cells from the cells to prepare a group of growing cells such as a cell sheet, a spheroid, or a colony with a homogenously thickness as the form after culturing.

### (Cell Culture Method)

The cell culture method of the present invention is provided with a step of inoculating the cells so as to be in contact with or held on one surface of the substrate forming the medical instrument of the present invention on which the convex-concave structure is formed, a step of culturing the cells to obtain cultured cells, and a step of adding a buffer solution such as phosphate-buffered physiological saline to the one surface to float the cultured cells from the one surface. Due to this, it is possible to release the cultured cells from the substrate without damage, and it is possible to culture the cells with efficient proliferation.

In the present invention, as a form of cell culturing, it is possible to culture floating cells in a standing state after inoculating the cells within the medical instrument. More specifically, it is possible to float and proliferate the cell culture of the present invention while forming a group as the cells.

This is realized by forming the surface of the substrate which contact or holds the cells of the fluorine-containing cyclic olefin polymer or the fluorine-containing cyclic olefin polymer composition, which has a hydrophobic surface property and a water contact angle of 70° or more in the present invention. Also, in order to keep the culture environment constant, air bubbles may be removed by tapping after inoculating, culturing may be carried out by applying vibration with consideration of the form and proliferation property of cells, culturing may be carried out while making the culture solution flow, or culturing may be carried out while stirring, without particular limitation. Among these, the culturing is favorably selected in consideration of the characteristics of the cells, the form of the apparatus, and the productivity. Regardless of the form, culturing the cells with the medical instrument according to the present invention makes it possible for the cells to proliferate while forming a group such as a cell sheet, a spheroid, or a colony while suppressing the attachment or close contact of the cells to the culturing instrument.

The culturing method is performed using medical instrument in which the substrate according to the present invention is processed into a container according to the various types of culturing methods. It is possible to carry out the cell culturing with a method, for example, such as standing culturing, rotating culturing, microcarrier culturing, gyratory culturing, spheroid culturing, culturing inside a gel, three-dimensional carrier culturing, and pressurizing circulation culturing. Among these, the method is favorably selected in consideration of the cell characteristics, the culture form, or the productivity and one method may be used individually or two or more types may be used together.

The temperature in these various types of culturing methods is preferably 35°C to 40°C, more preferably 36°C to 38°C, and particularly preferably 36.5°C to 37.5°C. In addition, the pressure is preferably 0.02 to 0.5 MPa, more preferably 0.05 to 0.3 MPa, and particularly preferably 0.08 to 0.2 MPa. Furthermore, the hydrogen ion index (pH) is preferably 8 to 6 and more preferably 7.5 to 6.5.

Examples of the sterilization method when using the medical instrument in the present invention as culturing instrument include wet sterilization involving dipping in alcohol or the like, gas sterilization by ethylene oxide, ultraviolet sterilization, radiation sterilization, autoclave sterilization by high temperature and high pressure water vapor, dry heat sterilization which is used for things which are not allowed to touch vapor directly, filtration sterilization which is suitable for a substrate which includes components which are unstable when heated, and the like. Among these, the sterilization is favorably selected in consideration of the process compatibility within a range which does not inhibit the effects of the present invention and two or more types of sterilization methods may be used in combination.

In addition, it is possible to select the type of culture medium which is used for the culturing according to the characteristics of the cells and the culture form regardless of the form such as liquid, gel, or solid (powder) . Examples thereof include a BME culture medium, an MEM culture medium, a DMEM culture medium, a 199 culture medium, an RPMI culture medium, a Ham' s F10 culture medium, a Ham's F12 culture medium, an MCDB 104, 107, 131, 151, 170, and 202 culture media, an RITC 80-7 culture medium, an MCDB 153 culture medium, and the like. These may be used individually or may be used in a combination of two or more types and moreover, may be used in a combination with a blood serum which is derived from animals such as humans, dogs, rats, mice, birds, pigs, or cows.

In addition, for example, collagen such as laminin-5, laminin-511, and laminin-521, the fluorine-containing cyclic olefin polymer of the present invention, the fluorine-containing cyclic olefin polymer composition, or the like may be applied on an inner wall of the substrate of the present invention or a part of one surface or all the surface which comes into contact with or holds the cells to be used according to the purpose of the cell culturing within a range which does not inhibit the effects of the present invention. The above may be used individually or in a combination of two or more types. In addition, in the present invention, it is possible to select a culture solution of cells or a buffer solution according to the type of the cells or the application of the cultured cells and a fluorescent pigment or cell hardening reagent may also be freely selected.

Furthermore, a buffer solution such as phosphate-buffered physiological saline, which is used for culturing or when separating cultured cells from the substrate by floating, may be any solution for which the hydrogen ion exponent (pH) does not change in the process of proliferating the cells and, generally, a single buffer solution may be used such as, for example, phosphate-buffered physiological saline, phosphate buffer solution, a hydrochloric acid buffer solution, an acetate buffer solution, a citric acid buffer solution, a boric acid buffer solution, a tartaric buffer solution, a tris buffer solution, a tris-hydrochloric acid buffer solution, an ethylene diamine tetraacetic acid buffer solution, a tris EDTA buffer solution, a tris acetate EDTA buffer solution, a tris boric acid EDTA buffer solution, a concentrated SSC buffer solution, a concentrated SSPE buffer solution, a sodium citrate buffer solution, a bicarbonate carbonate buffer solution, a sodium borate buffer solution, a maleic buffer solution, a CABS buffer solution, a piperidine buffer solution, a glycine buffer solution, a malic acid buffer solution, a formic acid buffer solution, a succinic acid buffer solution, an acetate buffer solution, a propionic acid buffer solution, a piperazine buffer solution, a pyridine buffer solution, a cacodylic acid buffer solution, a MES buffer solution, a histidine buffer solution, an ethanol amine buffer solution, an ADA buffer solution, a carbonate buffer solution, an ACES buffer solution, a PIPES buffer solution, an imidazole buffer solution, a bis-trispropane buffer solution, a BES buffer solution, a MOPS buffer solution, a HEPES buffer solution, a TES buffer solution, a MOPSO buffer solution, a MOBS buffer solution, a DIPSO buffer solution, a TAPSO buffer solution, a TEA buffer solution, a pyrophosphoric acid buffer solution, a HEPPSO buffer solution, a POPSO buffer solution, a tricine buffer solution, a hydrazine buffer solution, a glycylglycine buffer solution, an EPPS buffer solution, a bicin buffer solution, a HEPBS buffer solution, a TAPS buffer solution, an AMPD buffer solution, a TABS buffer solution, an AMPSO buffer solution, a taurine buffer solution, a CHES buffer solution, a glycine buffer solution, an ammonium hydroxide buffer solution, a CAPSO buffer solution, a methylamine buffer solution, a CAPS buffer solution, and the like individually, or a buffer solution which contains enzymes such as trypsin, pepsin, rennet, chymotrypsin, elastase, NADPH dehydrogenase, or NADH dehydrogenase may be used, preferably, a buffer solution containing phosphate-buffered physiological saline, phosphate-buffered physiological saline, a hydrochloric acid buffer solution, an acetate buffer solution, a citric acid buffer solution, a boric acid buffer solution, a tartaric buffer solution, a tris buffer solution, a tris-hydrochloric acid buffer solution, an ethylenediaminetetraacetic acid buffer solution, a tris EDTA buffer solution, a tris acetate EDTA buffer solution, a tris boric acid EDTA buffer solution, a concentrated SSC buffer solution, a concentrated SSPE buffer solution, a sodium citrate buffer solution, a bicarbonate carbonate buffer solution, a sodium borate buffer solution, a maleic buffer solution, and the like individually may be preferably used or a buffer solution which contains enzymes such as trypsin, pepsin, rennet, chymotrypsin, elastase, NADPH dehydrogenase, or NADH dehydrogenase may be used.

More preferably, phosphate-buffered physiological saline, phosphate-buffered physiological saline, a tris-hydrochloric acid buffer solution, an ethylenediaminetetraacetic acid buffer solution, a tris EDTA buffer solution, a tris acetate EDTA buffer solution, a tris boric acid EDTA buffer solution, a concentrated SSC buffer solution, a concentrated SSPE buffer solution, a sodium citrate buffer solution, a bicarbonate carbonate buffer solution, a sodium borate buffer solution, and the like may be used individually or a buffer solution which contains enzymes such as trypsin, pepsin, rennet, chymotrypsin, elastase, NADPH dehydrogenase, or NADH dehydrogenase may be used.

The present inventors invented a method in which, using the medical instrument of the present invention, for example, in the process of culturing animal cells including human cells and separating and collecting the cells, the cells are easily detached from a substrate in a state of floating naturally, for example, when a buffer solution such as phosphate-buffered physiological saline is added thereto.

According to the present invention, for the floating and detaching of the cells, it is possible to understand the phenomenon of floating and adhesion, for example, by observing the state of the interface between the cultured cells and the substrate. When cells were cultured for 7 days on a substrate formed of General Formula (1) of the present invention provided with a fine convex-concave structure serving as an anchorage of cells seen on the surface of a substrate used for ordinary spheroid culture and the interface between the cultured cells and the substrate was observed using a scanning electron microscope, even for cells grown on a substrate provided with a sufficiently small convex-concave structure necessary for anchorage formation, no anchor extending from the substrate was observed at the interface thereof, and when a buffer solution such as phosphate-buffered physiological saline was added to the cells cultured in this culture container, it was possible to detach the cells from the substrate while floating.

By culturing cells using the medical instrument formed of the fluorine-containing cyclic olefin polymer or the fluorine-containing cyclic olefin polymer composition of the present invention, it is possible to realize an excellent cell culture method in which cells proliferate efficiently while floating and forming a group such as a cell sheet, a spheroid, or a colony by floating and the proliferated growing cells are able to be floated and separated from the substrate without damaging the cells.

### Examples

Description will be given below of the present invention in the Examples; however, the present invention is not limited by these examples. Here, in the Examples, a polymer analysis value measuring method, preparation conditions and an analysis method for the substrate (film), a method for treating cells, a culturing instrument sterilization method, and a culture evaluation method will be described below.

### [Weight Average Molecular Weight (Mw) and Molecular Weight Distribution (Mw/Mn)]

Using gel permeation chromatography (GPC) under the following conditions, the weight average molecular weight (Mw) and the number average molecular weight (Mn) of the polymer which is dissolved in tetrahydrofuran (THF) or trifluoro toluene (TFT) were measured by calibrating the molecular weight according to a polystyrene standard.

Detection device: RI-2031 and 875-UV manufactured by JASCO Corporation or Model 270 manufactured by Viscotec, series-connected column: Shodex K-806M, 804, 803, and 802.5, column temperature: 40°C, flow amount: 1.0 ml/min, sample concentration: 3.0 to 9.0 mg/ml

### [Glass Transition Temperature]

Using DSC-50 manufactured by Shimadzu Corporation, the measuring sample was measured by heating at an increasing temperature speed of 10°C/min under a nitrogen atmosphere.

### [Water Contact Angle Measurement]

Using a solid content surface energy analysis apparatus CA-XE model manufactured by Kyowa Interface Science Co. , Ltd., on the basis of JIS R3257 (a wettability test method of a substrate glass surface), 2 *µ*l water droplets were dripped onto a substrate surface and the contact angle was measured within a minute after the water droplets came into contact with the substrate surface by a sessile drop method.

### [UV Curing]

For the curing of the applied film, the UV light imprint apparatus (X-100 U) manufactured by Scivax Corp., was used as a light source, and the culture film and the mold were cured by irradiation with LED light having a wavelength of 365 nm in a state with crimping or without crimping.

### [Measurement of Width (L1) Between Convexities of Culture Film]

Using a scanning electron microscope JSM-6701F (also referred to below as SEM) manufactured by JASCO Corporation, the width between convexities of nine arbitrary points was measured from the SEM photograph and the average value thereof was calculated.

### [Cell Type and Culture Solution]

Using a mouse embryo fibroblast (abbreviated below as BALB/3T3 cells), all the subculture and cell proliferation property tests were performed in a solution (also described below as a BALB/3T3 cell solution) which included 10% fetal bovine serum (Calf Bovine Serum, CBS), high glucose, and a D-MEM culture medium (containing L-glutamine, phenol red, and sodium pyruvate). In addition, all subculture and cell proliferation property tests of human skin fibroblasts (abbreviated below as Hs-68 cells) were carried out in the same manner.

### [Thawing Cells]

A frozen cell suspension of BALB/3T3 cells or Hs-68 cells was thawed by dipping in a 37°C water bath, 10% fetal bovine serum D-MEM culture medium which was cooled on ice was added to the thawed cell suspension, and centrifugal separation was carried out. After the centrifugal separation, the supernatant liquid was removed and the cell clusters loosened by tapping, then the 10% fetal bovine serum D-MEM culture medium which was kept in the 37°C water bath was added. After counting the number of the cells with a hemocytometer, preparing the cell suspension using the 10% fetal bovine serum D-MEM culture medium which was kept in the 37°C water bath, and inoculating the cells in a culture flask, culturing was carried out in a humidifying incubator.

### [Subculture of Cells]

The culture flask was taken out from the humidifying incubator, the culture medium was removed by an aspirator, Dulbecco PBS (-) which was kept in the 37°C water bath was added, and the supernatant liquid was removed by an aspirator. After performing the same operation again, a 0.025 w/v% trypsin-EDTA solution which was kept in the 37°C water bath was added and left to stand in the humidifying incubator, then the 10% fetal bovine serum D-MEM culture medium was added, the peeled off cells were collected and transferred into a centrifuge tube, the supernatant liquid was removed by carrying out centrifugal separation, the cell clusters were loosened by tapping, and then the 10% fetal bovine serum D-MEM culture medium was added. After counting the number of the cells with a hemocytometer, the cell suspension was prepared using the 10% fetal bovine serum D-MEM culture medium, the cells were inoculated in a culture flask, and culturing was carried out in a humidifying incubator.

### [Method for Sterilizing Cell Culturing instrument]

A culture film with a circular shape which was cut out to a diameter of 15 mm was placed in a TCPS multi-well plate (manufactured by Corning Incorporated) hole section, and dipping was carried out for 40 minutes to 1 hour by adding a 70% ethanol water solution thereto, and then the 70% ethanol water solution was removed and dipping was carried out in Dulbecco PBS (-) for 15 minutes to 40 minutes. Next, the PBS (-) was removed, the culturing instrument was turned over, the same operation was performed, and a sterilization process was carried out on the front and rear surfaces of the culturing instrument. After completing the sterilization process, drying was carried out in a clean bench overnight.

### [Preparation of Cell Suspension and Measurement of Cell Diameter]

The BALB/3T3 cells which were in an approximately 60% confluent state in a 25 cm² culture flask were peeled off by carrying out a treatment with 0.025 w/v% trypsin-EDTA by the same method as the subculture operation of the cells described above. The number of the cells was counted with a hemocytometer and cell suspensions of BALB/3T3 cells or Hs-68 cells at 7,300-7,600 cells/mL were prepared in a 10% calf serum D-MEM medium. In addition, in Example 10, a cell suspension in which Hs-68 cells were adjusted to 1,500 cells/mL was used.

From the average measurement of the cells per microscopic observation, the maximum diameter (L2) of the cells was 25 *µ*m. Alternatively, from the average measurement per cell Hs-68 cells prepared by the same method by microscopic observation, the maximum diameter (L2) of the cells was 20 *µ*m.

### [Confirmation of the Number of Cells Settled on Bottom Surface of Concave Portion]

BALB/3T3 cells or Hs-68 cells were inoculated on the surface to be cultured with cells with a convex-concave structure and placed in a humidifying incubator, after four hours, the samples were taken out from the humidifying incubator and observed using a microscope to count the number of cells at 30 arbitrary concave portions on the one surface of the substrate, and the number of cells settling on each bottom surface of a concave portion was shown as the average number (=number of cells/30).

### [Evaluation of Cell Proliferation Property]

The culture containers after 1, 3, 7 and 14 days after starting the culture were taken out from the humidifying incubator and the culture medium was removed, a mixed solution of 10% WST-8 (Cell Counting Kit-8)/10% fetal bovine serum D-MEM culture medium was added thereto, and incubating was carried out in a humidifying incubator for 3 hours. After that, the culture medium 200 *µ*l was transferred to a 96-well plate and the absorbance of the wavelength of 450 nm was measured by a plate reader (SPECTRA max PLUS 384, manufactured by Molecular Devices LCC.). The cell proliferation property of each culture container was confirmed according to the change in the absorbance over time.

### [Significance Test]

For various types of culture containers, nine samples in which cells were inoculated were prepared and cultured, the measurement results of the absorbance were analyzed according to the numeric value by Prism 6 for Windows 6.01 (produced by MDF Co., Ltd.), the average value of the results was calculated as the absorbance, and the standard deviation was given as a reference after ± and was the range of variation.

### [Fluorescence Microscope Observation]

The culture medium was removed from the container in which the cell culturing was carried out for the desired length of time, a 4% glutaraldehyde/phosphoric acid buffer solution was added and left to stand for 1 hour, and then the 4% glutaraldehyde/phosphoric acid buffer solution was removed. After that, cleansing was carried out using sterilizing water, cell nuclei and cytoskeletal proteins were dyed using an Image-iT Fixation/Permeabilization kit (manufactured by Life Sciences Corporation), and a sample which was used for fluorescence microscope observation was prepared. An all in one fluorescence microscope BZ-X700 (manufactured by Keyence Corporation) was used for the fluorescence microscope observation.

### [Production Example 1] Polymer 1

A tetrahydrofuran solution of Mo(N-2,6-Prⁱ₂C₆H₃) (CHCMe₂Ph) (OBu^{t})₂ (50 mg) was added to a tetrahydrofuran solution of 5,5,6-trifluoro-6-(trifluoromethyl)bicyclo[2.2.1]hepto-2-ene (100 g) and 1-hexene (0.268 g) and ring-opening metathesis polymerization was performed at 70°C. A hydrogenating reaction was carried out on the olefin portion of the obtained polymer at 160°C in the presence of palladium alumina (5 g) and a tetrahydrofuran solution of poly(1,1,2-trifluoro-2-trifluoromethyl-3,5-cyclopentylene ethylene) was obtained. The solution was filtered under pressure by a filter with a pore diameter of 5 *µ*m and a solution from which palladium alumina was removed was added to methanol, a white polymer was filtered, separated, and dried, and 99 g of a polymer 1 was obtained. The obtained polymer 1 contained a structural unit which is represented by General Formula (1). In addition, the hydrogenating ratio was 100%, the weight average molecular weight (Mw) was 83,000, the molecular weight distribution (Mw/Mn) was 1.73, and the glass transition temperature was 109°C.

### [Production Example 2] Culture Film 1

The polymer 1 which was synthesized in Production Example 1 was dissolved in methylisobutyl ketone at a concentration of 30% by mass, the solution was filtered under pressure by a filter with a pore diameter of 1 *µ*m and then filtered by a filter of 0.1 *µ*m to prepare a methylisobutyl ketone solution of the polymer 1. Subsequently, the methyl isobutyl ketone solution of polymer 1 was applied to a quartz mold having a lattice shape with a convex portion width of 100 *µ*m, a pattern pitch of 130 *µ*m, and a pattern depth of 40 *µ*m, uniformly coated with an applicator, and then peeled off after drying for 60 minutes at 140°C to obtain a culture film 1 with a lattice shape with a thickness of 80 *µ*m.

The width (L1) between convexities of the culture film 1 was 100 *µ*m, and the ratio (L1/L2) to the maximum diameter (L2 = 25 *µ*m) of BALB/3T3 cells was 4. In addition, the water contact angle of the culture film 1 was 126.0° (after 15 seconds).

### [Production Example 3] Culture Film 2

A methyl isobutyl ketone solution of the polymer 1 used in Production Example 2 was applied to a nickel mold having a lattice shape with a convex portion width of 500 *µ*m, a pattern pitch of 570 *µ*m, and a pattern depth of 70 *µ*m, uniformly coated with an applicator, then dried at 140°C for 60 minutes and peeled off to prepare a culture film 2 having a lattice shape with a thickness of 110 *µ*m.

The width (L1) between convexities of the culture film 2 was 500 *µ*m, and the ratio (L1/L2) to the maximum diameter (L2 = 25 *µ*m) of BALB/3T3 cells was 20. In addition, the water contact angle (after 15 seconds) was 134.1°.

### [Production Example 4] Culture film 3

A solution was prepared in which 20 g of a mixture with a mass ratio of 9/1 of 3-ethyl-3{[(3-ethyloxetan-3-yl)methoxy]methyl} oxetane as the photocurable compound (B) and 1,7-octadiene diepoxide and 0.8 g of Adekaoptomer SP-172 (produced by ADEKA Corporation) as a photo-curing initiator were added to 100 g of methyl isobutyl ketone solution in which the fluorine-containing cyclic olefin polymer 1 (A) synthesized in Production Example 1 was dissolved at 30% by mass concentration, the solution was filtered under pressure by a filter with a pore diameter of 1 *µ*m, and then filtered by a filter of 0.1 *µ*m to prepare a photocurable composition 1 (ratio of component (A) to component (B): [(A)/(B)=60/40]). Next, the photocurable composition 1 was uniformly coated on a quartz mold having a lattice shape with a convex portion width of 300 *µ*m, a pattern pitch of 350 *µ*m, and a pattern depth of 50 *µ*m using an applicator, then dried at 140°C for 30 minutes, ultraviolet light with a wavelength of 365 nm was irradiated from the rear surface of the coated surface at an accumulated light amount of 200 mJ/cm², and thereafter a culture film 3 having a lattice shape with a thickness of 90 *µ*m was prepared by being peeled from the mold.

The width (L1) between convexities of the culture film 3 was 300 *µ*m, and the ratio (L1/L2) to the maximum diameter (L2 = 25 *µ*m) of BALB/3T3 cells was 12. In addition, the water contact angle (after 15 seconds) was 128.1°.

### [Production Example 5] Culture Film 4

A solution was prepared in which 3 g of a mixture with a mass ratio of 9/1 of 3-ethyl-3{[(3-ethyloxetan-3-yl)methoxy]methyl} oxetane and 1,7-octadiene diepoxide as the photocurable compound (B) and 0.1 g of CPI-100P (produced by San-Apro Ltd) as a photo-curing initiator were added to 100 g of a cyclohexanone solution in which the fluorine-containing cyclic olefin polymer 1 (A) synthesized in Production Example 1 was dissolved at 30% by mass concentration, the solution was filtered under pressure by a filter with a pore diameter of 1 *µ*m, and then filtered by a filter of 0.1 *µ*m to prepare a photocurable composition 2 (ratio of component (A) to component (B) : [(A)/(B)=90/10]). Next, the photocurable composition 2 was uniformly coated on a nickel mold used in Production Example 3 and dried at 140°C for 50 minutes, ultraviolet light with a wavelength of 365 nm was irradiated from the rear surface of the coated surface at an accumulated light amount of 200 mJ/cm², and thereafter a culture film 4 having a lattice shape with a thickness of 105 *µ*m was prepared by being peeled from the mold.

The width (L1) between convexities of the culture film 4 was 500 *µ*m, and the ratio (L1/L2) to the maximum diameter (L2 = 25 *µ*m) of BALB/3T3 cells was 20. In addition, the water contact angle (after 15 seconds) was 129.4°.

### [Example 1]

The culture film 1 which was produced in Production Example 2 was sterilized and was placed on the bottom surface of a hole section of a 24-well TCPS multi-well plate with a pattern surface up and fixed in close contact with an O-ring (inner diameter of 11 mm) made of SUS with sterilizing grease (produced by Dow Corning Toray Co. , Ltd.), after which a BALB/3T3 cell solution (1 mL) was inoculated on a pattern surface of the culture film 1. The same operation was carried out nine times and nine samples were prepared as the number of samples. After that, a lid was put thereon, the samples were moved to a humidifying incubator, and culturing was started in sterilized air with a storage temperature of 37°C and a carbonic acid gas concentration of 5%. Subsequently, the sample after 4 hours was observed using a microscope, and the number of BALB/3T3 cells settled on the bottom surfaces of concave portions at 30 arbitrary places in the convex-concave structure of culture film 1 was counted and the average thereof was calculated. As a result, it was confirmed that 1.3 cells were present on each bottom surface of a concave portion.

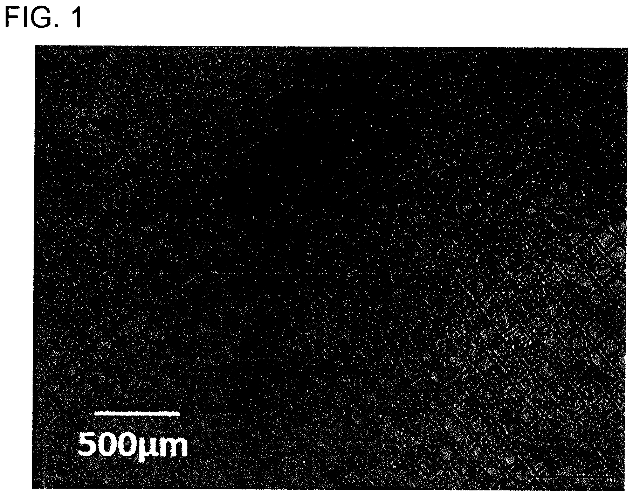

In the evaluation of the cell proliferation property by the WST-8 method, the absorbance was 0.31±0.02 after 1 day passed, 0.95±0.05 after 3 days passed, and 2.37±0.08 after 7 days passed. The absorbance increased in a straight line over the passing days and change was not seen in the proliferation property even after 7 days passed. In addition, when observing the cells which were cultured for 7 days using the microscope, the cells proliferated in a state of forming a group in the cell sheet in the thickness direction and floated in a state of keeping the group form in the cell sheet when phosphate-buffered physiological saline was added thereto (refer to FIG. 1).

### [Example 2]

The culture film 2 which was produced in Production Example 3 was sterilized and was fixed on a 24-well TCPS multi-well plate by the same method as Example 1 and a thawed BALB/3T3 cell solution (1 mL) was inoculated on the pattern surface of the culture film 2. The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%. Subsequently, the sample after 4 hours was observed using a microscope, and the number of BALB/3T3 cells settled on the bottom surfaces of concave portions at 30 arbitrary places in the convex-concave structure of culture film 2 was counted and the average thereof was calculated. As a result, it was confirmed that 26 cells were present on each bottom surface of a concave portion.

In the evaluation of the cell proliferation property by the WST-8 method, the absorbance was 0.24±0.02 after 1 day passed, 0.73±0.05 after 3 days passed, and 1.89±0.09 after 7 days passed. The absorbance increased in a straight line over the passing days and change was not seen in the proliferation property even after 7 days passed. In addition, when observing the cells which were cultured for 7 days using the microscope, the cells proliferated in a state of forming a group in the cell sheet in the thickness direction and floated in a state of keeping the group form in the cell sheet when phosphate-buffered physiological saline was added thereto.

### [Example 3]

The culture film 3 which was produced in Production Example 4 was sterilized and fixed on a 24-well TCPS multi-well plate by the same method as Example 1, and a thawed BALB/3T3 cell solution (1 mL) was inoculated on the pattern surface of the culture film 3. The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%. Subsequently, the sample after 4 hours was observed using a microscope, and the number of BALB/3T3 cells settled on the bottom surfaces of concave portions at 30 arbitrary places in the convex-concave structure of culture film 3 was counted and the average thereof was calculated. As a result, it was confirmed that 9.7 cells were present on each bottom surface of a concave portion.

In the evaluation of the cell proliferation property by the WST-8 method, the absorbance was 0.28±0.01 after 1 day passed, 0.89±0.02 after 3 days passed, and 2.26±0.07 after 7 days passed. The absorbance increased in a straight line over the passing days and change was not seen in the proliferation property even after 7 days passed. In addition, when observing the cells which were cultured for 7 days using the microscope, the cells proliferated in a state of forming a group in the cell sheet in the thickness direction and floated in a state of keeping the group form in the cell sheet when phosphate-buffered physiological saline was added thereto.

### [Example 4]

The culture film 4 which was produced in Production Example 5 was sterilized and fixed on a 24-well TCPS multi-well plate by the same method as Example 1, and a thawed BALB/3T3 cell solution (1 mL) was inoculated on a pattern surface of the culture film 4. The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%. Subsequently, the sample after 4 hours was observed using a microscope, and the number of BALB/3T3 cells settled on the bottom surfaces of concave portions at 30 arbitrary places in the convex-concave structure of culture film 4 was counted and the average thereof was calculated. As a result, it was confirmed that 26 cells were present on each bottom surface of a concave portion.

In the evaluation of the cell proliferation property by the WST-8 method, the absorbance was 0.24±0.03 after 1 day passed, 0.70±0.04 after 3 days passed, and 1.75±0.06 after 7 days passed. The absorbance increased in a straight line over the passing days and change was not seen in the proliferation property even after 7 days passed. In addition, when observing the cells which were cultured for 7 days using the microscope, the cells proliferated in a state of forming a group in the cell sheet in the thickness direction and floated in a state of keeping the group form in the cell sheet when phosphate-buffered physiological saline was added thereto.

### [Example 5]

97 g of the polymer 2 was obtained in the same manner as Production Example 1 except that the type of the fluorine-containing cyclic olefin monomer was changed to 5,6-difluoro-5-pentafluoroethyl-6-trifluoromethylbicyclo [2.2.1] hept-2-en. The obtained polymer 2 contained a structural unit represented by General Formula (1). In addition, the hydrogenation rate was 100%, the weight average molecular weight (Mw) was 91,000, the molecular weight distribution (Mw/Mn) was 1.93, and the glass transition temperature was 104°C.

Next, a culture film 5 having a lattice shape with a thickness of 85 *µ*m was prepared by the same method as in Production Example 2. The width (L1) between convexities of the culture film 5 was 100 *µ*m, and the ratio (L1/L2) to the maximum diameter (L2 = 25 *µ*m) of BALB/3T3 cells was 4. In addition, the water contact angle (after 15 seconds) was 128.1°.

In the culturing of BALB/3T3 cells carried out by the same method as in Example 1 with the culture film 5, when the sample after 4 hours was observed using a microscope, the BALB/3T3 cells settled to the bottom surfaces of concave portions of arbitrary 30 places of the convex-concave structure of the culture film 5 were counted, and the average value thereof was calculated, it was confirmed that 1.3 cells were present on each bottom surface of a concave portion.

In the evaluation of the cell proliferation property by the WST-8 method, the absorbance was 0.30±0.03 after 1 day passed, 0.99±0.03 after 3 days passed, and 2.28±0.11 after 7 days passed. The absorbance increased in a straight line over the passing days and change was not seen in the proliferation property even after 7 days passed. In addition, when observing the cells which were cultured for 7 days using the microscope, the cells proliferated in a state of forming a group in the cell sheet in the thickness direction and floated in a state of keeping the group form in the cell sheet when phosphate-buffered physiological saline was added thereto.

### [Example 6]

98 g of polymer 3 was obtained by the same method as in Production Example 1 except that the type of the fluorine-containing cyclic olefin monomer was changed to 5,6-difluoro-5-heptafluoro-iso-propyl-6-trifluoromethylbicyclo [2.2.1] hept-2-en. The obtained polymer 3 contained the structural unit represented by General Formula (1). In addition, the hydrogenation rate was 100%, the weight average molecular weight (Mw) was 142,000, the molecular weight distribution (Mw/Mn) was 1.40, and the glass transition temperature was 137°C.

Next, a culture film 6 having a lattice shape with a thickness of 103 *µ*m was prepared by the same method as in Production Example 2 except that the lattice-shaped nickel mold used in Production Example 3 was used as the mold. The width (L1) between convexities of the culture film 6 was 500 *µ*m, and the ratio (L1/L2) to the maximum diameter (L2 = 25 *µ*m) of BALB/3T3 cells was 20. In addition, the water contact angle (after 15 seconds) was 136.3°.

In the culturing of BALB/3T3 cells carried out by the same method as in Example 1 with the culture film 6, when the sample after 4 hours was observed using a microscope, the BALB/3T3 cells settled to the bottom surfaces of concave portions of arbitrary 30 places of the convex-concave structure of the culture film 6 were counted, and the average value thereof was calculated, it was confirmed that 26 cells were present on each bottom surface of a concave portion.

In the evaluation of the cell proliferation property by the WST-8 method, the absorbance was 0.21±0.03 after 1 day passed, 0.68±0.03 after 3 days passed, and 1.56±0.15 after 7 days passed. The absorbance increased in a straight line over the passing days and change was not seen in the proliferation property even after 7 days passed. In addition, when observing the cells which were cultured for 7 days using the microscope, the cells proliferated in a state of forming a group in the cell sheet in the thickness direction and floated in a state of keeping the group form in the cell sheet when phosphate-buffered physiological saline was added thereto.

### [Example 7]

After spin coating the photocurable composition 1 [(A)/(B) =60/40] prepared in Production Example 4 on a PET film and heating for 1 minute at 100°C, the PET film was covered such that the pattern surface of the quartz mold with a lattice shape used in Production Example 2 and the application surface of the photocurable composition 1 came into contact, and irradiated with UV light with a wavelength of 365 nm with a total light quantity of 200 mJ/cm² while pressing at a pressure of 0.3 MPa. Next, the PET film was separated from the quartz mold to produce a culture film 7 formed with a lattice shape on the surface of the PET film. The width (L1) between convexities of the culture film 7 was 100 *µ*m, and the ratio (L1/L2) to the maximum diameter (L2 = 25 *µ*m) of BALB/3T3 cells was 4. In addition, the water contact angle was 127.3° (after 15 seconds).

In the culturing of BALB/3T3 cells carried out by the same method as in Example 1 with the culture film 7, when the sample after 4 hours was observed using a microscope, the BALB/3T3 cells settled to the bottom surfaces of 30 arbitrary concave portions of the convex-concave structure of the culture film 7 were counted, and the average value thereof was calculated, it was confirmed that 1.3 cells were present on each bottom surface of a concave portion.

In the evaluation of the cell proliferation property by the WST-8 method, the absorbance was 0.27±0.01 after 1 day passed, 0.85±0.02 after 3 days passed, and 2.14±0.09 after 7 days passed. The absorbance increased in a straight line over the passing days and change was not seen in the proliferation property even after 7 days passed. In addition, when observing the cells which were cultured for 7 days using the microscope, the cells proliferated in a state of forming a group in the cell sheet in the thickness direction and floated in a state of keeping the group form in the cell sheet when phosphate-buffered physiological saline was added thereto.

### [Example 8]

The cell type was changed to Hs-68 cells, apart from this, the cells were thawed and subcultured and a suspension was prepared by the same method as the BALB/3T3 cells, and a cell suspension of 7, 500 cells/mL was prepared with a 10% fetal bovine serum D-MEM culture medium.

A culture film 9 was prepared by the same method as in Production Example 2 except that, in the preparation of the cultured film, the mold was changed to a quartz mold (convex portion width 300 *µ*m, pattern pitch 350 *µ*m, pattern depth 50 *µ*m) used in Production Example 4. The width (L1) between convexities of the culture film 9 was 300 *µ*m, and the ratio (L1/L2) to the maximum diameter (L2 = 20 *µ*m) of Hs-68 cells was 15. In addition, the water contact angle (after 15 seconds) was 124.2°.

Next, using the sterilized culture film 9, the thawed Hs-68 cell solution (1 mL) was inoculated by the same method as Example 1. The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%. When the sample after 4 hours was observed using a microscope, the number of Hs-68 cells settled at 30 arbitrary concave portion bottom surfaces of the convex-concave structure of the culture film 9 was counted, and the average value was calculated, it was confirmed that 9.7 cells were present on each bottom surface of a concave portion.

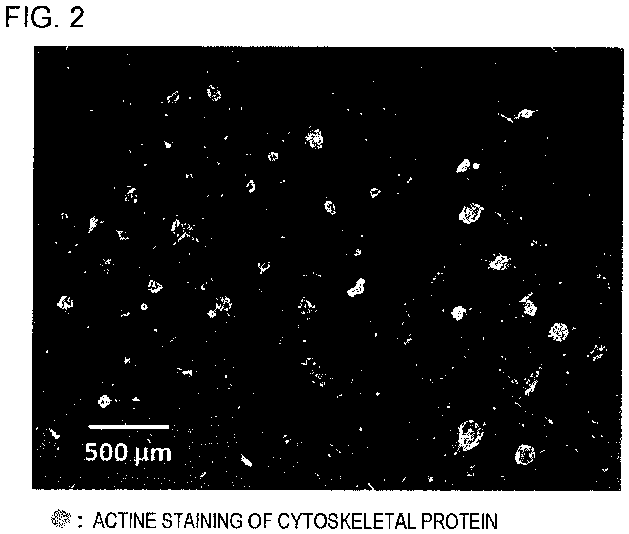

In the evaluation of the cell proliferation property by the WST-8 method, the absorbance was 0.27±0.05 after 1 day passed, 0.82±0.09 after 3 days passed, 1.69±0.11 after 7 days passed, and 3.21±0.13 after 14 days passed. The absorbance increased in a straight line over the passing days and change was not seen in the proliferation property even after 14 days passed. In addition, when observing the cells which were cultured for 14 days using the microscope, the cells proliferated in a state (refer to FIG. 2) of forming a group in a spheroid or colony form and floated in a state of a spheroid or colony form when trypsin-containing phosphate-buffered physiological saline was added thereto.

When evaluating the drug-metabolizing enzyme activity of these cultured cells in NADPH dehydrogenase, it was understood that almost 100% had a drug metabolizing system enzyme activity similar to living cells. Furthermore, when the autofluorescence was observed, it was confirmed that 100% of the cultured cells were living cells in the same manner.

### [Example 9]

Using the sterilized culture film 1 (L1/L2=5), the thawed Hs-68 cell solution (1 mL) was inoculated by the same method as Example 8. The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%. When the sample after 4 hours was observed using a microscope, the number of Hs-68 cells settled at 30 arbitrary concave portion bottom surfaces of the convex-concave structure of the culture film 1 was counted, and the average value was calculated, it was confirmed that 1.3 cells were present on each bottom surface of a concave portion.

In the evaluation of the cell proliferation property by the WST-8 method, the absorbance was 0.28±0.04 after 1 day passed, 1.83±0.05 after 3 days passed, and 1.79±0.10 after 7 days passed. The absorbance increased in a straight line over the passing days and change was not seen in the proliferation property even after 7 days passed. In addition, when observing the cells which were cultured for 7 days using the microscope, the cells proliferated in sheet form in a state of forming a group in a cell sheet in the thickness direction and floated in a state of keeping the sheet form when trypsin-containing phosphate-buffered physiological saline was added thereto.

When evaluating the drug-metabolizing enzyme activity of these cultured cells in NADPH dehydrogenase, it was understood that almost 100% had a drug metabolizing system enzyme activity similar to living cells. Furthermore, when the autofluorescence was observed, it was confirmed that 99.8% of the cultured cells were living cells in the same manner.

### [Example 10]

Using the sterilized culture film 9 (L1/L2 = 15), a thawed Hs-68 cell solution (1 mL) prepared by the same method as in Example 8 except that the number of cells was changed to 1, 500 cells/mL was inoculated. The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%. When the sample after 4 hours was observed using a microscope, the number of Hs-68 cells settled at 30 arbitrary concave portion bottom surfaces of the convex-concave structure of the culture film 9 was counted, and the average value was calculated, it was confirmed that 1.9 cells were present on each bottom surface of a concave portion.

In the evaluation of the cell proliferation property by the WST-8 method, the absorbance was 0.06±0.01 after 1 day passed, 0.17±0.09 after 3 days passed, and 0.36±0.12 after 7 days passed. The absorbance increased in a straight line over the passing days and change was not seen in the proliferation property even after 7 days passed. In addition, when observing the cells which were cultured for 7 days using the microscope, the cells proliferated in a state of forming a spheroid or a colony and floated in a state of keeping the spheroid or a colony form when trypsin-containing phosphate-buffered physiological saline was added thereto.

When evaluating the drug-metabolizing enzyme activity of these cultured cells in NADPH dehydrogenase, it was understood that almost 100% had a drug metabolizing system enzyme activity similar to living cells. Furthermore, when the autofluorescence was observed, it was confirmed that 100% of the cultured cells were living cells in the same manner.

### [Example 11]

Using the sterilized culture film 3 (L1/L2=15), the thawed Hs-68 cell solution (1 mL) was inoculated by the same method as Example 8. The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%. When the sample after 4 hours was observed using a microscope, the number of Hs-68 cells settled at 30 arbitrary concave portion bottom surfaces of the convex-concave structure of the culture film 3 was counted, and the average value was calculated, it was confirmed that 9.6 cells were present on each bottom surface of a concave portion.

In the evaluation of the cell proliferation property by the WST-8 method, the absorbance was 0.21±0.04 after 1 day passed, 0.74±0.09 after 3 days passed, and 1.60±0.11 after 7 days passed. The absorbance increased in a straight line over the passing days and change was not seen in the proliferation property even after 7 days passed. In addition, when observing the cells which were cultured for 7 days using the microscope, the cells proliferated in sheet form in a state of forming a group in a cell sheet in the thickness direction and floated in a state of keeping the sheet form when trypsin-containing phosphate-buffered physiological saline was added thereto.

When evaluating the drug-metabolizing enzyme activity of these cultured cells in NADPH dehydrogenase, it was understood that almost 100% had a drug metabolizing system enzyme activity similar to living cells. Furthermore, when the autofluorescence was observed, it was confirmed that 99.9% of the cultured cells were living cells in the same manner.

### [Example 12]

Using the sterilized culture film 6 (L1/L2=25) prepared in Example 6, the thawed Hs-68 cell solution (1 mL) was inoculated by the same method as Example 8. The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%. When the sample after 4 hours was observed using a microscope, the number of Hs-68 cells settled at 30 arbitrary concave portion bottom surfaces of the convex-concave structure of the culture film 6 was counted, and the average value was calculated, it was confirmed that 26 cells were present on each bottom surface of a concave portion.

In the evaluation of the cell proliferation property by the WST-8 method, the absorbance was 0.23±0.05 after 1 day passed, 0.65±0.08 after 3 days passed, and 1.55±0.11 after 7 days passed. The absorbance increased in a straight line over the passing days and change was not seen in the proliferation property even after 7 days passed. In addition, when observing the cells which were cultured for 7 days using the microscope, the cells proliferated in a state of forming a spheroid and floated in a state of keeping the spheroid form when trypsin-containing phosphate-buffered physiological saline was added thereto.

When evaluating the drug-metabolizing enzyme activity of these cultured cells in NADPH dehydrogenase, it was understood that almost 100% had a drug metabolizing system enzyme activity similar to living cells. Furthermore, when the autofluorescence was observed, it was confirmed that 100% of the cultured cells were living cells in the same manner.

### [Example 13]

A film of polymer 1 having a thickness of 120 *µ*m was prepared from the methyl isobutyl ketone solution of the polymer 1 used in Production Example 2 by a solution casting method. Next, the film of the polymer 1 and the pattern surface of the quartz mold (convex portion width 300 *µ*m, pattern pitch 350 *µ*m, pattern depth 50 *µ*m) used in Production Example 4 were brought into contact with each other on a heating plate, left on the heating plate, heated to 150°C, thermocompression bonding was carried out at 10 MPa and held for 5 seconds as it was. After cooling to 70°C, the mold was detached to prepare the culture film 10. The width (L1) between convexities of the culture film 10 was 300 *µ*m, and the ratio (L1/L2) to the maximum diameter (L2 = 20 *µ*m) of Hs-68 cells was 15. The water contact angle (after 15 seconds) was 124.9°.

Next, thawed Hs-68 cell solution (1 mL) was inoculated using the sterilized culture film 10 by the same method as in Example 1. The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%. When the sample after 4 hours was observed using a microscope, the number of Hs-68 cells settled at 30 arbitrary concave portion bottom surfaces of the convex-concave structure of the culture film 10 was counted, and the average value was calculated, it was confirmed that 9.8 cells were present on each bottom surface of a concave portion.

In the evaluation of the cell proliferation property by the WST-8 method, the absorbance was 0.26±0.04 after 1 day passed, 0.87±0.03 after 3 days passed, and 1.70±0.10 after 7 days passed. The absorbance increased in a straight line over the passing days and change was not seen in the proliferation property even after 7 days passed. In addition, when observing the cells which were cultured for 7 days using the microscope, the cells proliferated in a state of forming a spheroid or a colony and floated in a state of keeping the spheroid or a colony form when trypsin-containing phosphate-buffered physiological saline was added thereto.

When evaluating the drug-metabolizing enzyme activity of these cultured cells in NADPH dehydrogenase, it was understood that almost 100% had a drug metabolizing system enzyme activity similar to living cells. Furthermore, when the autofluorescence was observed, it was confirmed that 100% of the cultured cells were living cells in the same manner.

### [Example 14]

The bottom surface of a concave portion of the 6-well TCPS multi-well plate (manufactured by Corning) was cut out, a film 1 in which the width (L1) between convexities produced in Production Example 2 was 100 *µ*m and the ratio (L1/L2) to the maximum diameter (L2 = 20 *µ*m) of human embryonic fibroblasts was 5 was affixed to the bottom surfaces of the concave portions of all six holes from the rear surface of the container, and a cell culture container was prepared and sterilized with ethanol.

Next, thawed Hs-68 cell solution was inoculated by the same method as in Example 8, except that the amount of the cell solution was changed to 10 mL. The same operation was carried out nine times and nine samples were prepared as the number of samples. After that, the lid was moved and moved to the incubator, and culturing was started in sterilized air having a storage temperature of 37°C and a carbonic acid gas concentration of 5%. When the sample after 4 hours was observed using a microscope, the number of Hs-68 cells settled at 30 arbitrary concave portion bottom surfaces of the convex-concave structure of the culture film 1 was counted, and the average value was calculated, it was confirmed that 1.3 cells were present on each bottom surface of a concave portion.

In the evaluation of the cell proliferation property by the WST-8 method, the absorbance was 0.29±0.01 after 1 day passed, 0.83±0.01 after 3 days passed, and 1.79±0.05 after 7 days passed. The absorbance increased in a straight line over the passing days and change was not seen in the proliferation property even after 7 days passed. In addition, when observing the cells which were cultured for 7 days using the microscope, the cells proliferated in a state of forming a group in the thickness direction in a sheet form, and floated in a state of keeping the sheet form when trypsin-containing phosphate-buffered physiological saline was added thereto.

When evaluating the drug-metabolizing enzyme activity of these cultured cells in NADPH dehydrogenase, it was understood that almost 100% had a drug metabolizing system enzyme activity similar to living cells. Furthermore, even when the autofluorescence was observed, since almost no fluorescence was observed, it was confirmed that 100% of the cultured cells were living cells in the same manner.

### [Reference Example 1]

A methyl isobutyl ketone solution of the polymer 1 used in Production Example 2 was applied to a nickel mold having a pillar shape with a diameter of 150 nm and a pitch of 250 nm, uniformly coated using an applicator, and then dried at 140°C for 60 minutes and separated to prepare a culture film 11 having a hole shape having a thickness of 50 *µ*m. The water contact angle (after 15 seconds) of the culture film 11 was 124.3°.

Next, using the sterilized culture film 11, the film was fixed in a 24-well TCPS multi-well plate by the same method as in Example 1, and a thawed BALB/3T3 cell solution (1 mL) was inoculated on the pattern surface of the culture film 11. The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%.

In the evaluation of the cell proliferation property by the WST-8 method, the absorbance was 0.10±0.02 after 1 day passed, 0.34±0.03 after 3 days passed, and 0.70±0.09 after 7 days passed. The absorbance increased in a straight line over the passing days and change was not seen in the proliferation property even after 7 days passed. In addition, when observing the cells which were cultured for 7 days using the microscope, the cells proliferated in a state of forming a group in the cell sheet in the thickness direction and floated in a state of keeping the group form in the cell sheet when phosphate-buffered physiological saline was added thereto.

Furthermore, the culture solution of the BALB/3T3 cells cultured for 7 days was removed, dehydrated, and dried to prepare a sample for SEM observation, and when the state of the interface between the cells and the culture film 11 was observed, no filamentous anchorage formation extending from the cell so as to be entangled with a hole (concave portion) having the maximum diameter of 150 nm of the pattern surface the culture film 11 was observed.

### [Comparative Example 1]

A thawed BALB/3T3 cell solution (1 mL) was inoculated on the bottom surface of the hole of a γ-ray sterilized 24-well TCPS multi-well plate (manufactured by Corning, water contact angle after 15 seconds, 46.1°). The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%. When the sample after 4 hours was observed using a microscope and the bottom surface of the multi-well plate was observed, sparsely scattered BALB/3T3 cells were observed, some of which settled in the form of aggregates, and the settling was in a non-uniform state with respect to the culturing surface as a whole.

In the evaluation of the cell proliferation property by the WST-8 method, the absorbance was 0.27+0.03 after 1 day passed, 1.08+0.11 after 3 days passed, and 1.51±0.23 after 7 days passed. A tendency for the increase ratio of the absorbance to gradually attenuate over the passing days was seen and the extent of the cell proliferation was reduced as the days passed. In addition, when observing the cells which were cultured for 7 days using the microscope, the cells proliferated in sheet form in a planar state with uneven thickness in the thickness direction and did not change in form or float even when phosphate-buffered physiological saline was added thereto.

### [Comparative Example 2]

The culturing of BALB/3T3 cells was started by the same method as in Example 1 using ethanol-sterilized Apel™ film (produced by Mitsui Chemicals, Inc.). When the sample after 4 hours was observed using a microscope, sparsely scattered BALB/3T3 cells were observed, some of which settled in the form of aggregates, and the settling was in a non-uniform state with respect to the culturing surface as a whole.

In the evaluation of the cell proliferation property by the WST-8 method, the absorbance was 0.11±0.01 after 1 day passed, 1.22±0.10 after 3 days passed, and 1.71±0.19 after 7 days passed. A tendency for the increase ratio of the absorbance to gradually attenuate over the passing days was seen and the extent of the cell proliferation was reduced as the days passed. In addition, when observing the cells which were cultured for 7 days using the microscope, the cells proliferated in sheet form in a planar state with uneven thickness in the thickness direction and did not change in form or float even when phosphate-buffered physiological saline was added thereto.

### [Comparative Example 3]

49 g of a polymer 4 was obtained by the same method as in Production Example 1 except that the monomer described in Production Example 1 was changed to 5-methyl-bicyclo [2.2.1] hept-2-ene and the solvent was changed to cyclohexane. In addition, the hydrogenation ratio was 100%, the weight average molecular weight (Mw) was 129, 000, the molecular weight distribution (Mw/Mn) was 2.26, and the glass transition temperature was 67°C.

Next, a cyclohexanone solution in which polymer 4 was dissolved at 30% by mass was applied to a glass substrate and dried at 150°C for 3 hours to prepare a film having a thickness of 75 *µ*m. Thereafter, a lattice-shaped culture film 8 having a thickness of 73 *µ*m was produced by a heat melting compression bonding nano-imprinting method using the lattice-shaped quartz mold used in Production Example 2. The width (L1) between convexities of the culture film 8 was 100 *µ*m, and the ratio (L1/L2) to the maximum diameter (L2 = 25 *µ*m) of BALB/3T3 cells was 4. In addition, the water contact angle (after 15 seconds) was 101.2°.

In the culturing of BALB/3T3 cells carried out by the same method as in Example 1 with the culture film 8, when the sample after 4 hours was observed using a microscope, the number of BALB/3T3 cells settled at 30 arbitrary concave portion bottom surfaces of the convex-concave structure of the culture film 4 was counted, and the average value was calculated, it was confirmed that 1.4 cells were present on each bottom surface of a concave portion.

In the evaluation of the cell proliferation property by the WST-8 method, the absorbance was 0.07±0.01 after 1 day passed, 0.15±0.02 after 3 days passed, and 0.18±0.11 after 7 days passed. The extent of the cell proliferation was reduced as the days passed. In addition, when observing the cells which were cultured for 7 days using the microscope, the cells proliferated in sheet form in a planar state with uneven thickness in the thickness direction and did not change in form or float even when phosphate-buffered physiological saline was added thereto.

### [Comparative Example 4]

Except for changing the cell suspension to an Hs-68 cells solution (10 mL), culturing was started in sterilized air in an incubator with a storage temperature of 37°C and a carbonic acid gas concentration of 5% in the same manner as Comparative Example 1.

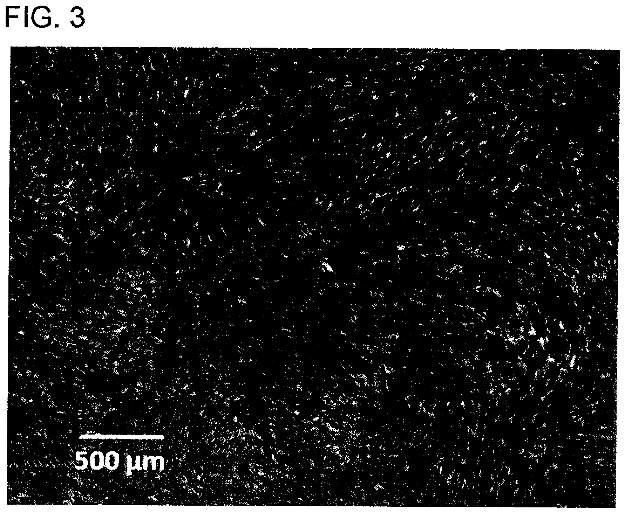

In the evaluation of the cell proliferation property by the WST-8 method, the absorbance was 0.33±0.06 after 1 day passed, 1.49±0.11 after 3 days passed, and 3.10±0.13 after 7 days passed. A tendency for the absorbance to gradually attenuate over the passing days was seen and the extent of the cell proliferation was reduced as the days passed. In addition, when observing the cells which were cultured for 7 days using the microscope, the cells proliferated in sheet form and did not change in form or float even when trypsin-containing phosphate-buffered physiological saline was added thereto (refer to FIG. 3).

Furthermore, when the cells which were cultured for 7 days were stained with a fluorescence reagent and the form of the cell nucleus and the cytoskeletal protein were observed under a fluorescence microscope, a blue fluorescent light-emitting cell nucleus and a green fluorescent light-emitting cytoskeletal protein were observed in a fluorescent emission distribution in a sheet form spread two-dimensionally and it was not possible to confirm group formation in the cell sheet in which the cells were overlapped in the thickness direction.

When the drug metabolizing system enzyme activity of these cultured cells in NADPH dehydrogenase was evaluated, it was found that approximately 0% had a drug metabolizing system enzyme activity similar to that of the living cells. Furthermore, in the autofluorescence observation, measurement was not possible of γ-ray sterilized 24-well TCPS multi-well plate by fluorescence absorption.

### [Comparative Example 5]

A thawed BALB/3T3 cell solution (1 mL) was inoculated on the pattern surface of bottom surfaces of holes of a sterilized 24-hole nano-culture plate (manufactured by Scivax Corporation, the water contact angle (after 15 seconds) was 125°, L1/L2 = 0.16) having a pattern pitch of 4 *µ*m and having a hexagonal close-packed array. The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%.

When the sample after 4 hours was observed using the microscope and 30 arbitrary concave portion bottom surfaces of the convex-concave structure were observed, settled BALB/3T3 cells were not contained in even one concave portion.

In the evaluation of the cell proliferation property by the WST-8 method, the absorbance was 0.07±0.03 after 1 day passed, 0.09±0.02 after 3 days passed, and 0.10±0.09 after 7 days passed. A tendency for the increase ratio of the absorbance to gradually attenuate over the passing days was seen and the extent of the cell proliferation was reduced as the days passed. In addition, when observing the cells which were cultured for 7 days using the microscope, the cells proliferated by forming a group of spheroids in the thickness direction, and the sizes and shapes thereof were uneven and did not change in form or float even when phosphate-buffered physiological saline was added thereto.

Furthermore, when the culture solution of BALB/3T3 cells cultured for 7 days was removed, dehydrated, and dried to prepare a sample for SEM observation, and the state of the interface between the cells and, when the nano-culture plate was observed, a pattern was observed in which the anchorage of the cell was formed so as to be entangled with the concave portion of the pattern surface of the nano-culture plate.

### [Comparative Example 6]

A cell suspension of Hs-68 cells was inoculated by the same method as Example 8 on the pattern surface of the bottom surface of the hole of a sterilized 6-well spheroid-forming culture container (IWAKI & Co. , Ltd. , L1/L2 = 25) having a hole shape of 500 *µ*m in bore diameter. The same operation was carried out nine times and nine samples were prepared as the number of samples. After that, a lid was put thereon, the samples were moved to an incubator, and culturing was started in sterilized air with a storage temperature of 37°C and a carbonic acid gas concentration of 5%.

When the sample after 4 hours was observed using a microscope, the number of Hs-68 cells settled at 30 arbitrary concave portion bottom surfaces of the convex-concave structure was counted, and the average value was calculated, it was confirmed that 25 cells were present on each bottom surface of a concave portion.

In the evaluation of the cell proliferation property by the WST-8 method, the absorbance was 0.08±0.01 after 1 day passed, 0.11±0.04 after 3 days passed, and 0.15±0.06 after 7 days passed. In addition, when observing the cells which were cultured for 7 days using the microscope, the cells proliferated by forming a group of spheroids in the thickness direction, but the sizes and shapes thereof were uneven and did not change in form or float even when phosphate-buffered physiological saline was added thereto.

This application claims priority based on Japanese Patent Application No. 2015-070868 filed on March 31, 2015, the disclosure of which is incorporated herein in its entirety.

The present invention includes the following aspects.
<1>
   Medical instrument comprising a substrate, in which the substrate has one surface with which cells are brought into contact, the one surface of the substrate which comes into contact with the cells is provided with a convex-concave structure formed of a fluorine-containing cyclic olefin polymer containing a structural unit represented by General Formula (1) below, a ratio (L1/L2) of a width (L1) between convexities formed of the convex-concave structure and a diameter (L2) of each cell is 1 to 100, and the cells do not adhere to the one surface provided with the convex-concave structure. 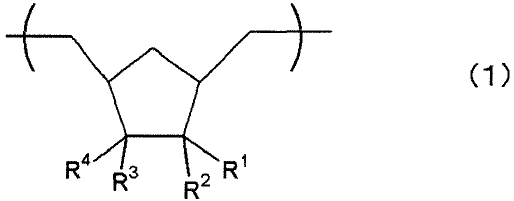 (In Formula (1), at least one of R¹ to R⁴ is fluorine, an alkyl group with 1 to 10 carbon atoms which contains fluorine, an alkoxy group with 1 to 10 carbon atoms which contains fluorine, or an alkoxyalkyl group having 2 to 10 carbon atoms which contains fluorine, and, when R¹ to R⁴ are a group not containing fluorine, R¹ to R⁴ are selected from hydrogen, an alkyl group with 1 to 10 carbon atoms, an alkoxy group with 1 to 10 carbon atoms, or an alkoxyalkyl group having 2 to 10 carbon atoms, R¹ to R⁴ may be the same as or different from each other, and R¹ to R⁴ may be bonded to each other to form a cyclic structure.)
<2>
   The medical instrument according to <1>, in which the width between convexities of the convex-concave structure of the one surface with which the cells are brought into contact is 10 *µ*m to 1,000 *µ*m.
<3>
   The medical instrument according to <1> or <2>, in which the water contact angle of the one surface with which the cells are brought into contact is 70° to 160°.
<4>
   The medical instrument according to any one of <1> to <3>, in which the one surface of the substrate is formed of a fluorine-containing cyclic olefin polymer composition including a fluorine-containing cyclic olefin polymer, a photocurable compound, and a photo-curing initiator.
<5>
   The medical instrument according to <4>, in which a mass ratio (fluorine-containing cyclic olefin polymer/photocurable compound) of the fluorine-containing cyclic olefin polymer and the photocurable compound in the fluorine-containing cyclic olefin polymer composition is 99.9/0.1 to 50/50.
<6>
   The medical instrument according to any one of <1> to <5>, which is used for culturing the cells in contact with the one surface.
<7>
   The medical instrument according to <6>, in which, in the cell culturing, the cells float and proliferate while forming a group.
<8>
   The medical instrument according to <6> or <7>, in which the cultured cells are floated using phosphate-buffered physiological saline and detached from the one surface.
<9>
   A fluorine-containing cyclic olefin polymer used, in medical instrument in which cells are brought into contact with one surface of a substrate having a convex-concave structure, for forming the one surface, the fluorine-containing cyclic olefin polymer composition comprising a structural unit represented by General Formula (1) below. 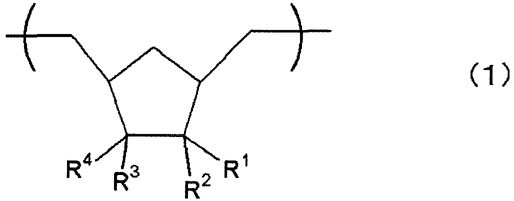 (In Formula (1), at least one of R¹ to R⁴ is fluorine, an alkyl group with 1 to 10 carbon atoms which contains fluorine, an alkoxy group with 1 to 10 carbon atoms which contains fluorine, or an alkoxyalkyl group having 2 to 10 carbon atoms which contains fluorine, and, when R¹ to R⁴ are a group not containing fluorine, R¹ to R⁴ are selected from hydrogen, an alkyl group with 1 to 10 carbon atoms, an alkoxy group with 1 to 10 carbon atoms, or an alkoxyalkyl group having 2 to 10 carbon atoms, R¹ to R⁴ may be the same as or different from each other, and R¹ to R⁴ may be bonded to each other to form a cyclic structure.)
<10>
   A fluorine-containing cyclic olefin polymer composition used for forming the one surface of medical instrument for bringing cells into contact with one surface of a substrate having a convex-concave structure, the fluorine-containing cyclic olefin polymer composition comprising a fluorine-containing cyclic olefin polymer containing a structural unit represented by General Formula (1) below, a photocurable compound, and a photo-curing initiator. 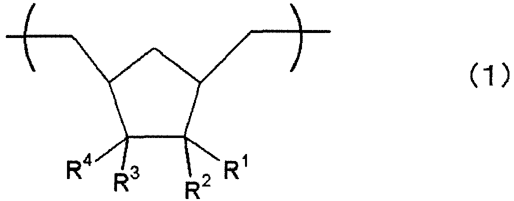 (In Formula (1), at least one of R¹ to R⁴ is fluorine, an alkyl group with 1 to 10 carbon atoms which contains fluorine, an alkoxy group with 1 to 10 carbon atoms which contains fluorine, or an alkoxyalkyl group having 2 to 10 carbon atoms which contains fluorine, and, when R¹ to R⁴ are a group not containing fluorine, R¹ to R⁴ are selected from hydrogen, an alkyl group with 1 to 10 carbon atoms, an alkoxy group with 1 to 10 carbon atoms, or an alkoxyalkyl group having 2 to 10 carbon atoms, R¹ to R⁴ may be the same as or different from each other, and R¹ to R⁴ may be bonded to each other to form a cyclic structure.)
<11>
   A cell culture method comprising a step of inoculating the cells on the one surface of the medical instrument according to any one of <1> to <8> so as to contact the one surface; a step of culturing the cells to obtain cultured cells; and a step of adding a phosphate-buffered physiological saline solution to the one surface to float the cultured cells from the one surface.

## Claims

1. Medical instrument comprising:
a substrate using a fluorine-containing cyclic olefin polymer containing a structural unit represented by General Formula (1),
wherein the substrate has one surface where the substrate comes into contact with cells, and the substrate is provided with a convex-concave structure on the one surface, a ratio (L1/L2) of a width (L1) between convexities formed by the convex-concave structure and a maximum diameter (L2) of inoculated cells per cell in the cells is 1 to 300, and the cells do not adhere to or attach to the one surface provided with the convex-concave structure and the medical instrument promotes cell proliferation. 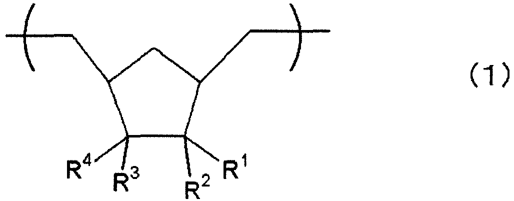 (in Formula (1), at least one of R¹ to R⁴ is fluorine, an alkyl group with 1 to 10 carbon atoms which contains fluorine, an alkoxy group with 1 to 10 carbon atoms which contains fluorine, or an alkoxyalkyl group with 2 to 10 carbon atoms which contains fluorine, and, when R¹ to R⁴ are groups which do not contain fluorine, R¹ to R⁴ are selected from hydrogen, an alkyl group with 1 to 10 carbon atoms, an alkoxy group with 1 to 10 carbon atoms, or an alkoxyalkyl group with 2 to 10 carbon atoms, R¹ to R⁴ may be the same as or different from each other, and R¹ to R⁴ may be bonded to each other to form a cyclic structure.)

2. The medical instrument according to claim 1,
wherein the L1/L2 is 1 to 100.

3. The medical instrument according to claim 1 or 2,
wherein a width between convexities of the convex-concave structure is 10 *µ*m to 1,000 *µ*m.

4. The medical instrument according to any one of claims 1 to 3,
wherein the one surface has a water contact angle of 70° to 160°.

5. The medical instrument according to any one of claims 1 to 4,
wherein the one surface is formed by a fluorine-containing cyclic olefin polymer composition including the fluorine-containing cyclic olefin polymer, a photocurable compound, and a photo-curing initiator.

6. The medical instrument according to claim 5,
wherein a mass ratio (fluorine-containing cyclic olefin polymer/photocurable compound) of the fluorine-containing cyclic olefin polymer and the photocurable compound in the fluorine-containing cyclic olefin polymer composition is from 99.9/0.1 to 50/50.

7. The medical instrument according to any one of claims 1 to 6, which is used for culturing cells in contact with the one surface.

8. The medical instrument according to claim 7,
wherein the cultured cells from the cells float and proliferate while forming a group of growing cells selected from cell sheets, spheroids, or colonies.

9. The medical instrument according to claim 7 or 8,
wherein a group of growing cells is liberated by a buffer solution and detached from the one surface.

10. A fluorine-containing cyclic olefin polymer used, in medical instrument in which cells are brought into contact with one surface of a substrate having a convex-concave structure, for forming the one surface, the fluorine-containing cyclic olefin polymer comprising:
a structural unit represented by General Formula (1) below. 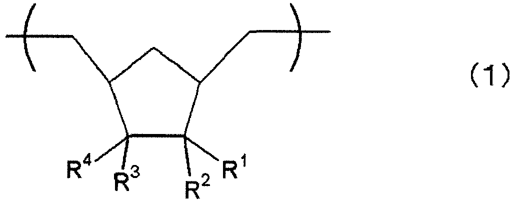 (in Formula (1), at least one of R¹ to R⁴ is fluorine, an alkyl group with 1 to 10 carbon atoms which contains fluorine, an alkoxy group with 1 to 10 carbon atoms which contains fluorine, or an alkoxyalkyl group with 2 to 10 carbon atoms which contains fluorine, and, when R¹ to R⁴ are groups which do not contain fluorine, R¹ to R⁴ are selected from hydrogen, an alkyl group with 1 to 10 carbon atoms, an alkoxy group with 1 to 10 carbon atoms, or an alkoxyalkyl group with 2 to 10 carbon atoms, R¹ to R⁴ may be the same as or different from each other, and R¹ to R⁴ may be bonded to each other to form a cyclic structure.)

11. A fluorine-containing cyclic olefin polymer composition used, in medical instrument in which cells are brought into contact with one surface of a substrate having a convex-concave structure, for forming the one surface, the fluorine-containing cyclic olefin polymer composition comprising:
a fluorine-containing cyclic olefin polymer containing a structural unit represented by General Formula (1) below;
a photocurable compound; and
a photo-curing initiator. 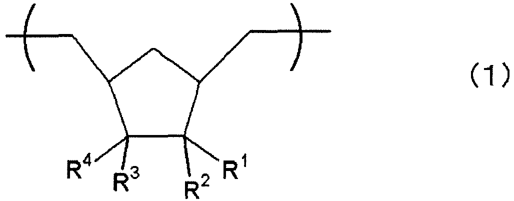 (in Formula (1), at least one of R¹ to R⁴ is fluorine, an alkyl group with 1 to 10 carbon atoms which contains fluorine, an alkoxy group with 1 to 10 carbon atoms which contains fluorine, or an alkoxyalkyl group with 2 to 10 carbon atoms which contains fluorine, and, when R¹ to R⁴ are groups which do not contain fluorine, R¹ to R⁴ are selected from hydrogen, an alkyl group with 1 to 10 carbon atoms, an alkoxy group with 1 to 10 carbon atoms, or an alkoxyalkyl group with 2 to 10 carbon atoms, R¹ to R⁴ may be the same as or different from each other, and R¹ to R⁴ may be bonded to each other to form a cyclic structure.)

12. A cell culture method comprising:
a step of inoculating cells so as to contact one surface of the medical instrument according to any one of claims 1 to 9;
a step of culturing the cells to obtain cultured cells; and
a step of adding a buffer solution to the one surface to float cultured cells from the one surface.
